(19) Europäisches Patentamt
European Patent Office
Office européen des brevets

(11) Numéro de publication: **0 086 139**
**A2**

(12) # DEMANDE DE BREVET EUROPEEN

(21) Numéro de dépôt: 83400187.7

(22) Date de dépôt: 27.01.83

(51) Int. Cl.³: C 12 N 15/00
C 12 N 9/02, C 12 N 1/20
C 12 N 7/00, A 61 K 37/50
A 23 B 7/14, C 11 D 3/386

(30) Priorité: 01.02.82 FR 8201574

(43) Date de publication de la demande:
17.08.83 Bulletin 83/33

(84) Etats contractants désignés:
AT BE CH DE FR GB IT LI LU NL SE

(71) Demandeur: TRANSGENE S.A.
95 rue Saint-Lazare
F-75009 Paris(FR)

(72) Inventeur: Zukowski, Mark M.
4, quai Mathiss
F-67000 Strasbourg(FR)

(72) Inventeur: Gaffney, Dairena
20 Kenvinside Gardens
Glasgow G20 6(GB)

(72) Inventeur: Speck, Denis
1 rue de Saverne
F-67200 Eckbolsheim(FR)

(72) Inventeur: Lecocq, Jean-Pierre
6, rue du Cham du Feu
F-67116 Reichstett(FR)

(74) Mandataire: Warcoin, Jacques et al,
Cabinet Régimbeau 26, avenue Kléber
F-75116 Paris(FR)

(54) **Nouveaux vecteurs d'expression de la catéchol 2,3-oxygénase, enzymes obtenues et leurs applications.**

(57) La présente invention concerne un plasmide vecteur caractérisé en ce qu'il comporte au moins :
- le gène *xylE* codant pour la synthèse de la catéchol 2,3-oxygénase, et
- un promoteur de l'expression fonctionnelle du gène *xylE* dans une bactérie gram ⁺.

Les bactéries transformées à l'aide de ces plasmides peuvent être utilisées pour le clonage d'ADN et également pour la synthèse de la catéchol 2,3-oxygénase.

EP 0 086 139 A2

Croydon Printing Company Ltd

# NOUVEAUX VECTEURS D'EXPRESSION DE LA CATECHOL 2,3-OXYGENASE, ENZYMES OBTENUES ET LEURS APPLICATIONS.

La présente invention concerne de façon générale le clonage et l'expression du gène codant pour la catéchol 2,3-oxygénase dans différents microorganismes, notamment des bactéries gram + telles que Bacillus subtilis et le phage M13.

Plus particulièrement, la présente invention concerne de nouveaux vecteurs, et en particulier des plasmides vecteurs, de clonage et d'expression, ainsi que leurs applications.

Enfin, la présente invention concerne l'application dans différents domaines de l'enzyme obtenue, à savoir la catéchol 2,3-oxygénase.

Cette enzyme est impliquée dans le schéma de dégradation du toluène, notamment par les souches de Pseudomonas.

Il est déjà connu que certaines souches de Pseudomonas ont la propriété de dégrader le toluène.

Ces souches possèdent, en effet, un plasmide appelé TOL ou pWWO (117 kb) codant pour une série d'enzymes qui transforment progressivement le toluène en dérivé directement assimilable par la cellule selon un schéma rappelé ci-après :

SCHEMA 1

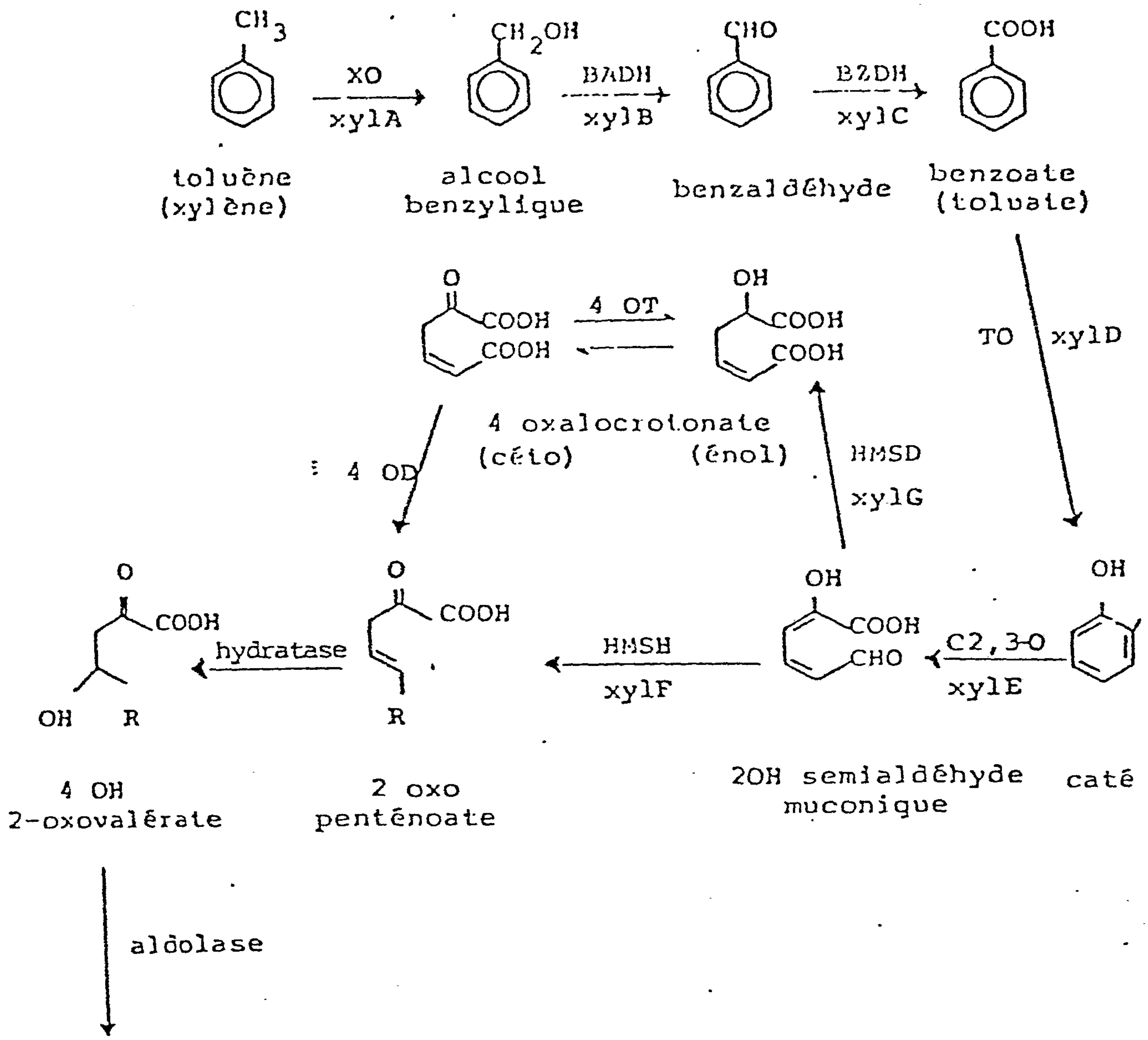


pyruvate $CH_3COCOOH$ + aldéhyde $RCH_2CHO$

XO : xylène oxygénase ; BADH : alcool benzylique déhydrogénase ;
BZDH : benzaldéhyde déhydrogénase ; TO : toluate/benzoate oxygéna
C 2,3-O : catéchol 2,3-oxygénase ; HMSD : hydroxymuconique
semialdéhyde déhydrogénase ; 4-OT : 4-oxalocrotonate tautomérase
HMSH : hydroxymuconique semialdéhyde hydrolase ;
4-OD : 4-oxalocrotonate décarboxylase.

La catéchol 2,3-oxygénase (C 2,3-O) (EC n° 1.13.11.2), transforme le catéchol en semialdéhyde muconique selon la réaction suivante :

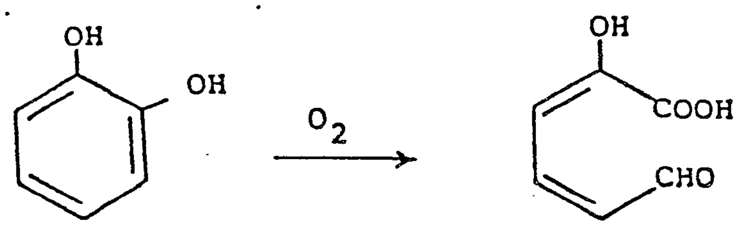

L'activité d'une telle enzyme peut être facilement mise en évidence par un test coloré puisque le catéchol se présente sous forme incolore, alors que le produit de dégradation, à savoir le semialdéhyde muconique, présente une couleur jaune.

On appellera ci-après et par abréviation la catéchol 2,3-oxygénase : C 2,3-O, et le gène codant pour la synthèse de cette enzyme : <u>xylE</u> ou bien gène C 2,3-O.

Compte tenu du fait que, comme cela a été indiqué précédemment, cette enzyme dégrade le catéchol incolore pour le transformer en semialdéhyde jaune, il y a là une possibilité d'obtenir un test coloré permettant de trier facilement un grand nombre de colonies bactériennes pour déterminer lesquelles ont été transformées par un plasmide déterminé portant le gène <u>xylE</u> ou bien dans lesquelles, au contraire, le gène <u>xylE</u> a été inactivé.

Certains auteurs (réf. 6,9,27) ont réussi à faire exprimer le gène <u>xylE</u> dans <u>Escherichia coli</u> qui est une bactérie gram - comme les <u>Pseudomonas</u> dont provient le gène <u>xylE</u>.

L'expression du gène xylE dans les souches E. coli ou Pseudomonas présente un inconvénient au niveau industriel car ces souches sont potentiellement pathogènes pour l'homme. Il est donc difficile, sans un grand nombre de précautions, d'envisager la production de catéchol oxygénase au stade industriel à partir de ces bactéries.

La bactérie la plus utilisée au stade industriel est Bacillus subtilis qui est une bactérie gram + dans laquelle il est souvent difficile de faire s'exprimer des gènes hétérospécifiques.

Il était donc intéressant de mettre au point des plasmides comportant le gène xylE qui s'exprime dans des bactéries gram +,et notamment B. subtilis, afin de permettre une production industrielle de C 2,3-O. En outre, l'expression de la C 2,3-O dans B. subtilis présente l'avantage de donner accès à un test coloré chez cette bactérie, pour laquelle un tel test n'existe pas, alors qu'il s'agit d'une bactérie de grande importance industrielle.

Dans le cas des phages, en particulier du phage M13, lequel est utilisé couramment sous forme notamment de phage M13 mp7 (commercialisé par la société Bethesda Research Laboratories, Inc. et par la société P.L. Laboratories) à titre de moyen pour séquencer des ADN, le remplacement du test coloré utilisant le colorant X-gal (5-bromo-4-chloro-3-indolyl-β-D-galactoside) par un test coloré mettant en oeuvre le catéchol constituerait certainement un avantage considérable, tant sur le plan de l'efficacité que sur le plan du prix, le catéchol étant environ 5 000 fois moins cher que le colorant X-gal.

Enfin, l'enzyme catéchol 2,3-oxygénase ainsi préparée présente un intérêt industriel important, tant par son action sur le catéchol lui-même que sur les dérivés dudit catéchol.

Les applications selon la présente invention de l'enzyme catéchol 2,3-oxygénase peuvent être regroupées autour de deux centres d'intérêt essentiels :

Tout d'abord l'industrie alimentaire ; en effet, les fruits contiennent une quantité importante de catéchols qui, sous l'action de la lumière et de l'oxygène, sont transformés par les polyphénoloxydases en composés colorés qui sont responsables du brunissement des fruits.

L'utilisation de la C 2,3-O pour transformer le catéchol des fruits en aldéhyde en empêchant ainsi l'action des polyphénoloxydases peut donc permettre d'éviter le brunissement des fruits.

Pour cette utilisation, il est bien entendu possible d'utiliser l'enzyme purifiée, mais on peut envisager également d'utiliser directement la bactérie contenant ladite enzyme, en particulier lorsqu'il s'agit déjà de bactéries d'intérêt alimentaire telles que Lactobacillus ou Streptococcus lactiques.

L'enzyme C 2,3-O est également utilisable dans l'industrie pharmaceutique et cosmétique. En effet, il est bien connu que le catéchol et certains dérivés du catéchol constituent des allergènes puissants, le plus connu, le 3-pentadécylcatéchol de formule :

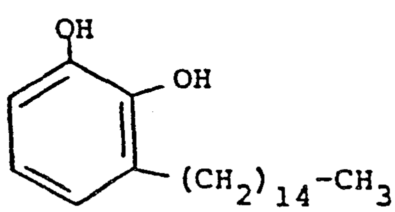

étant présent dans différentes espèces de Toxicodendron tels que le "poison ivy" et le "poison oak" ou "arbre poison" lesquels sont responsables d'une allergie de contact touchant presque 50 % des américains.

Or, on a constaté que la C 2,3-O dégradait le cycle aromatique de dérivés apparentés au catéchol et devait ainsi en diminuer très fortement l'allerginicité.

On peut envisager également dans le domaine pharmaceutique d'utiliser le C 2,3-O pour modifier la structure des catécholamines (adrénaline, noradrenaline, DOPA, DOPAMINE,...).

Dans le domaine cosmétique, la C 2,3-O peut également être utilisée pour limiter la présence de composés de type catéchol qui apparaît lors de la dégradation de certaines bases cosmétiques.

Pour ces deux types d'application alimentaire et cosméto-pharmaceutique, il est particulièrement intéressant de pouvoir préparer la C 2,3-O à partir de Bacillus subtilis.

L'intérêt de Bacillus subtilis comme souche hôte est qu'il s'agit, à la différence d'Escherichia coli, d'un organisme non pathogène qui n'est relié en aucune manière à une affection quelconque humaine ou animale.

C'est pourquoi l'utilisation de B. subtilis ou des produits de son métabolisme au stade industriel est autorisée, à la différence d'E. coli.

En outre B. subtilis est parmi les bactéries gram + celle qui est la plus connue et qui a été le plus largement étudiée.

Enfin, B. subtilis est une bactérie largement utilisée dans l'industrie pour la production d'enzymes, notamment d'amylases et de protéases dont les conditions de culture industrielles sont bien connues.

Pour ce faire, la présente invention concerne des plasmides vecteurs caractérisés en ce qu'ils comportent au moins :

- le gène xylE codant pour la synthèse de la catéchol 2,3-oxygénase, et
- un promoteur assurant l'expression fonctionnelle du gène xylE dans une bactérie gram +.

Le promoteur de l'expression fonctionnelle du gène xylE peut être obtenu à partir d'une séquence d'ADN chromosomique d'une bactérie gram +, mais il a également été mis en évidence que de tels promoteurs pouvaient être obtenus à partir d'autres matériaux, notamment à partir de plasmides ou de bactériophages. Ainsi, on peut utiliser des promoteurs provenant de l'ADN chromosomique de diverses souches de Bacillus, B. subtilis, B. licheniformis, B. pumilus, mais on peut utiliser des promoteurs provenant de l'ADN chromosomique de bactéries gram - telles que E. coli. De même, il est possible d'utiliser des promoteurs provenant de plasmides, en particulier de plasmides de bactéries gram +, notamment ceux se répliquant dans B. subtilis tels que pUB110. Enfin, il est possible d'utiliser des promoteurs provenant de bactériophages, notamment des bactériophages de bactéries gram + tels que le bactériophage $\phi 29$.

Il est évident que les promoteurs envisagés dans le cadre de la présente invention peuvent être divers et s'adapter à chaque type particulier de souche gram +, Bacillus, Streptococcus, Lactobacillus, Staphylococcus et Corynebacterium par exemple.

Comme cela a été indiqué précédemment, l'intérêt de ces plasmides vecteurs étant en priorité de pouvoir cloner des gènes déterminés dans Bacillus subtilis, ou bien d'obtenir l'enzyme C 2,3-O par culture de B. subtilis, on recherchera de préférence un promoteur de l'expression fonctionnelle du gène xylE dans une séquence d'ADN chromosomique de Bacillus et plus particulièrement de Bacillus subtilis.

Lorsque le plasmide vecteur selon la présente invention doit servir de vecteur de clonage, il est particulièrement intéressant qu'il comporte au moins un site unique de restriction situé à un endroit tel

que l'insertion d'une séquence d'ADN dans ce site conduise à l'inactivation du gène xylE. Ainsi ce site peut être situé, soit dans le gène xylE, ou bien en aval du promoteur et en amont du gène xylE. Une telle inactivation peut facilement être mise en évidence par une pulvérisation de catéchol sur l'ensemble des colonies et sélection des colonies qui, bien que transformées, ne se colorent pas en jaune du fait que le gène xylE n'est pas exprimé.

Parmi les sites de restriction uniques présentant un intérêt, il faut citer plus particulièrement un site de restriction BamH1 qui peut être aisément introduit, comme cela sera démontré dans les exemples pour les plasmides dérivés de pTG402 tels que pTG403, pTG404, pTG405,pTG406, pTG407, pTG408,pTG409 et pTG410.

La présente invention concerne également des souches de Bactéries gram +, en particulier les souches de Bacillus subtilis, transformées par les plasmides selon la présente invention. Parmi les bactéries gram + utilisables, il faut citer également les genres Lactobacillus, Streptococcus , Staphylococcus et Corynebacterium.

La présente invention concerne également un autre type de vecteur qui est un phage dérivé du phage M13, caractérisé en ce que son ADN comporte au moins le gène xylE codant pour la synthèse de la catéchol 2,3-oxygénase.

De préférence, l'ADN de ce phage comportera au moins un site unique de restriction placé de façon que l'insertion d'une séquence ADN dans ce site inactive l'expression du gène xylE. De préférence,

il existera plusieurs sites uniques de restriction (appelés sites "de clonage multiple") qui joueront la même fonction.

L'intérêt du phage M13 est le suivant ; un dérivé de ce coliphage filamenteux est utilisé pour cloner et séquencer les ADN, il s'agit en particulier du phage M13 mp7 développé par le Dr. J. Messing et qui est distribué par Bethesda Research Laboratories et PL Labs sous forme d'ensemble pour cloner et séquencer les ADN.

Ce phage comporte un site de clonage multiple dans lequel il est possible d'insérer de nombreux fragments de restriction tels que des fragments EcoR1, BamH1, AccI, Hind11, PstI et Sal1.

Ainsi, lorsque l'on introduit dans le milieu de croissance d'une souche et du phage M13 mp7 de l'isopropyl-β-D-galactoside et du 5-bromo-4-chloro-3-indolyl-β-D-galactoside, l'isopropyl-β-D-galactoside induit une partie du gène lacZ sur le phage M13 qui, lorsqu'il est complémenté par la partie finale du gène porté sur le facteur F' dans la même cellule, conduit à un phénotype β-galactosidase positif. Ceci a pour effet de faire virer l'indicateur 5-bromo-4-chloro-3-indolyl-β-D-galactoside (colorant X-gal) au bleu.

L'insertion d'un fragment d'ADN au niveau du site de clonage multiple conduit à l'inactivation de la complémentation α de la β-galactosidase, dans ces conditions l'indicateur coloré ne change pas de couleur. Toutefois, il faut remarquer que cet indicateur est très cher, en effet, il faut compter environ 3 francs français d'indicateur par boîte de Pétri, et comme dans chaque

expérience on est obligé d'utiliser un grand nombre de boîtes, ceci conduit parfois à des dépenses très importantes.

L'utilisation du phage selon la présente invention permet d'avoir recours à un indicateur beaucoup moins onéreux, le catéchol, et, en outre, de pouvoir utiliser le phage M13 dans différentes souches sans être dépendants d'une complémentation et donc de souches particulières contenant le gène lacZΔ M15 comme dans le cas du phage M13 mp7.

Rappelons que pour son utilisation dans le séquençage, il faut hybrider avec le DNA du phage un oligonucléotide d'une quinzaine de bases complémentaires à la région du DNA très proche du site d'insertion.

Indépendamment des vecteurs décrits précédemment en eux-mêmes, la présente invention concerne également leur application au clonage de gènes dans des bactéries ainsi que les bactéries transformées par ces plasmides et leur utilisation pour la préparation de la catéchol 2,3-oxygénase.

Enfin, la présente invention concerne l'application de la catéchol 2,3-oxygénase obtenue, notamment par la mise en oeuvre de bactéries selon l'invention, à titre d'additif alimentaire, en particulier d'agent de conservation dans l'industrie alimentaire et comme conservateur en cosmétique, et à titre de principe actif en thérapeutique pour le traitement et la prévention des allergies provoquées notamment par le catéchol et des composés dérivés du catéchol.

Enfin, la catéchol 2,3-oxygénase peut être utilisée dans des compositions de décontamination, notamment pour le linge ayant pu entrer en contact avec l'urushiol ou ses dérivés. Ce type de composition permet d'éliminer les risques d'allergie provoquée par un contact récurant de la peau avec des tissus ayant été en contact avec des espèces de Toxicodendron, comme cela a été indiqué précédemment.

Il s'agit de compositions de décontamination de surface, caractérisées en ce qu'elles comportent une base détergente et une enzyme, la catéchol 2,3-oxygénase.

Compte tenu des conditions de stabilité de l'enzyme, ces compositions auront, de préférence, un pH compris entre 6 et 10 et contiendront de préférence un agent de stabilisation tel que l'acétone.

Grâce à la présente invention qui permet de préparer la C 2,3-O à partir de B. subtilis, qui est un microorganisme déjà utilisé pour préparer des protéases entrant dans la préparation de détergents tels que les lessives par exemple, on peut envisager de préparer des souches transformées productrices à la fois de protéase et de C 2,3-O.

Parmi les compositions de décontamination, outre les lessives, on peut prévoir des bains moussants ou des lotions diverses, ces formes de mise en oeuvre n'étant pas limitatives. Enfin, on a constaté que la C 2,3-O était compatible avec un nombre important de compositions détergentes existant, ce qui évite de formuler des nouvelles compositions.

Bien entendu, la C 2,3-O peut être mise en forme comme le sont déjà les protéases utilisées dans les lessives, par exemple sous forme de granules tel que cela est décrit notamment dans les brevets français 1 520 948 ou 2 119 480. Les brevets mentionnés précédemment donnent également des informations sur les éléments entrant dans la préparation des lessives.

Les propriétés de la catéchol 2,3-oxygénase ont été étudiées sur des extraits bruts acellulaires bactériens.

L'extrait bactérien s'est révélé capable d'oxyder, outre le catéchol, le 4-méthylcatéchol, avec l'apparition d'un produit jaune présentant 2 pics d'absorption : 326 et 378 nm.

Pour mesurer l'activité enzymatique des souches obtenues on utilise une technique spectrophotométrique en mesurant l'accroissement d'absorbance à 375 mm dû à l'accumulation dans le milieu réactionnel du semialdéhyde.

Protocole

Dans une cuve spectro :

- 2,9 ml de tampon phosphate 0,1 M, pH 6,8,
- 0,1 ml d'extrait bactérien,
- 10 µl d'une solution de catéchol 0,02 M,

cinétique effectuée à 30°C et mesurée sur une minute. L'appareil (Uvikon 810/820) est doté d'une unité de calcul qui fournit par l'intermédiaire d'une imprimante les résultats suivants :

- DO pour chaque mesure effectuée,
- Δ DO entre chaque mesure,
- vitesse exprimée en DO/min.,
- calcul du coefficient de corrélation qui permet de vérifier la bonne linéarité de la courbe.

Une mesure est effectuée toutes les 6 secondes pendant une minute (10 points).

L'activité enzymatique est exprimée en mU/mg de protéines. Une mU correspond a une nanomole de produit formé en une minute à 30°C dans les conditions précédemment décrites.

Stabilité de l'enzyme en fonction de la température

L'extrait est traité 10 minutes à différentes températures et l'activité est mesurée dans les conditions décrites précédemment.

| Température | 30°C | 55°C | 60°C | 65°C |
|---|---|---|---|---|
| Activité de la C 2,3-O (%) | 100 | 84 | 18 | 3 |

L'extrait est traité, cette fois, à 55°C pendant différents temps.

| Durée (minutes) | 0 | 10 | 20 | 30 |
|---|---|---|---|---|
| Activité de la C 2,3-O (%) | 100 | 84 | 39 | 24 |

D'après ces résultats, l'enzyme montre une bonne résistance vis-à-vis de la température et les résultats ci-dessous vont confirmer sa bonne stabilité.

L'extrait est placé à température ambiante, dilué dans différents tampons à une concentration en protéines de 0,32 mg/ml.

| Durée | 0 | 19 h | 2,5 jours | 6 jours |
|---|---|---|---|---|
| Activité (%) | | | | |
| .Après stockage dans l'eau | 100 | 54 | 7 | 6 |
| .Phosphate 0,1 M pH 6,8 | 100 | 84 | 63 | 13 |
| .Phosphate 0,1 M pH 7,5 - 10 % acétone | 100 | 93 | 81 | 61 |

Ces résultats montrent l'effet stabilisant de l'acétone qui est d'ailleurs utilisée dans la technique d'extraction par plusieurs auteurs (réf. 28, 29)

Activité en fonction du pH

Des essais enzymatiques effectués dans du phosphate 0,1 M à des pH variables ont montré un optimum d'activité à 6,75.

| pH | 5,6 | 6 | 6,5 | 6,75 | 7 | 7,5 | 8 | 8,5 | 8,9 |
|---|---|---|---|---|---|---|---|---|---|
| Activité (%) | 10 | 40 | 78 | 100 | 93 | 80 | 61 | 52 | 49 |

Température optimale

En outre, il a été démontré que l'activité maximum se situe à 45°C.

| Température d'incubation (°C) | 27 | 30 | 35 | 40 | 45 | 50 | 53 |
|---|---|---|---|---|---|---|---|
| Activité (%) | 75 | 100 | 125 | 162 | 200 | 131 | 119 |

On remarquera que l'activité à la température de référence (30°C) représente la moitié de l'activité maximum.

Parmi les compositions selon la présente invention incorporant la catéchol 2,3-oxygénase, il faut citer plus particulièrement les compositions pour application topique externe dans le domaine cosmétique et médicamenteux. Les bases utilisables dans ce type de compositions sont connues de l'homme de l'art et ne constituent pas des caractéristiques de la présente invention. Toutefois, on pourra tenir compte de l'effet stabilisant de l'acétone ou d'autres solvants protecteurs pour la C 2,3-O et du pH optimum d'activité situé entre 6,5 et 7,5, pour la réalisation des compositions selon l'invention.

Lorsque la C 2,3-O est utilisée comme conservateur dans l'industrie alimentaire, en particulier pour éviter le brunissement des fruits, il est possible d'utiliser l'enzyme purifiée ou un extrait bactérien, mais il est possible d'envisager dans certains cas d'utiliser la bactérie elle-même, en particulier lorsque le produit alimentaire contenant les fruits est déjà un produit de fermentation bactérienne.

Les exemples ci-après permettront de mettre en évidence d'autres caractéristiques et avantages de la présente invention. De façon générale, les références bibliographiques sont rassemblées en fin de description et ne sont rappelées que par leurs numéros dans le corps de la description.

Les figures ci-annexées permettront de mieux comprendre certains aspects de la présente invention.

Sur ces figures :

- la figure 1 représente une carte de restriction du plasmide pTG200,
- la figure 2 représente les cartes de restriction des plasmides pTG206, pHV33 et pTG402,
- la figure 3 représente la séquence de nucléotide du gène xylE et du site de fixation des ribosomes,
- la figure 4 représente la stratégie utilisée pour séquencer le gène xylE,
- la figure 5 représente un diagramme d'électrophorèse de différents extraits bactériens contenant la C 2,3-O.

Dans un premier temps, le gène *xylE*, qui est entièrement compris dans un des fragments *XhoI* du plasmide pWWO, a été cloné sur despetits plasmides de façon à disposer d'une source d'ADN de *xylE* facile à recloner pour la suite des expériences sans avoir à sélectionner tous les fragments de restriction de pWWO qui est un très grand plasmide.

En outre, ces petits plasmides ont permis de préparer de grandes quantités de catéchol 2,3-oxygénase pour l'étude de cette enzyme.

EXEMPLE 1 - Clonage et expression du gène codant pour la catéchol 2,3-oxygénase dans Escherichia coli

1a) Souches et plasmides

Le plasmide pWWO a été extrait d'une souche de *Pseudomonas putida*.

Le plasmide vecteur est le plasmide pKT230 (réf. 6) qui code pour la résistance à la kanamycine et à la streptomycine. Ce plasmide possède un site *XhoI* unique dans le gène de résistance à la kanamycine.

Le plasmide pBR322 a également été utilisé comme vecteur de clonage.

Les souches hôtes sont toutes des dérivés d'*E. coli* K12. SK 1592 est *gal thi sbc* B15 *end* A *hsd* R4 $hsdM^+$ (réf. 25). BZ18 $rk^-$ $mk^+$ *gal lac* y SuII. W3110 trpOE $met^-$ (réf. 26).

1b) Procédé

Afin de cloner le gène *xylE* dans pKT230, l'ADN de pWWO a été coupé avec l'enzyme de restriction *XhoI* et ligaturé avec un fragment *XhoI* de l'ADN du vecteur traité à la phosphatase alcaline.

Des études précédentes ont démontré que ce gène xylE est entièrement situé dans le fragment de restriction I (XhoI) du plasmide pWWO et dans un sous-fragment BamHI-XhoI de ce fragment. (6,9)

La souche SK 1592 a été transformée à l'aide des plasmides obtenus et les colonies résistant à la streptomycine ont été triées grâce à une pulvérisation de catéchol.

En effet, les souches contenant l'enzyme catéchol 2,3-oxygénase virent au jaune lorsqu'elles sont mises en présence de catéchol, compte tenu de la production du semialdéhyde 2-hydroxymuconique (réf.14 ).

Les recombinants contenant l'ensemble du gène xylE mais qui ont perdu une portion de l'ADN du plasmide vecteur sont isolés. La carte de restriction du plasmide pTG200 est représentée sur la figure 1 ; le trait épais représente le fragment ADN de pWWO.

Ce plasmide pTG200 est utilisé comme source d'ADN codant pour le gène xylE dans les expériences suivantes :

Le fragment BamHI-XhoI de pTG200 a été cloné dans les sites BamHI et SalI du plasmide pBR322. Le plasmide dérivé est décrit dans le tableau I.

## TABLEAU I

## PLASMIDES PORTANT LE GENE xylE

| | |
|---|---|
| SK1592/pTG200 | pWWO coupé avec XhoI et cloné dans pKT230 aussi coupé par XhoI. Délétion d'une partie de l'ADN pKT230 proche du site XhoI. |
| BZ18/pTG206 | pBR322 coupé avec BamHI-SalI et intégration du fragment BamHI-XhoI de pTG200 ($Ap^R$ $Tc^S$) |

$Ap^R$ : Résistance à l'ampicilline
$Tc^S$ : Sensibilité à la tétracycline

Le niveau d'expression de la catéchol 2,3-oxygénase dans chacune des souches contenant les plasmides recombinants obtenus ont également été comparés.

La concentration en protéines est déterminée par la technique de Lowry (réf. 11)

Les résultats mesurés sont rassemblés dans le tableau 2.

TABLEAU 2

ACTIVITE SPECIFIQUE EN CATECHOL 2,3-OXYGENASE
DANS DIFFERENTES SOUCHES EN PHASE EXPONENTIELLE DE CROISSANCE
(activité en mU/mg)

| Souche hôte / Plasmide | Croissance 30°C | Croissance 37°C |
|---|---|---|
| BZ18 / pBR322 (controle) | | < 0,5 |
| SK1592 / pTG200 | | $1,7 \times 10^3$ |
| BZ18 / pTG206 | | $4,0 \times 10^4$ |
| p.putida/pWWO | $1,3 \times 10^2$ | |

Comme cela ressort du tableau 2, la souche obtenue présente un degré élevé de catéchol 2,3-oxygénase et ainsi peut être considérée comme utilisable pour la synthèse de cette enzyme.

Une carte de restriction partielle de pTG206 est représentée sur la figure 2A sur laquelle figurent, outre les sites de restriction, la localisation du gène xylE et le gène $A_p^R$. La partie en trait plus épais représente la partie utilisée dans la construction de pTG402 (voir exemples suivants).

On a, ainsi, obtenu un ensemble de plasmides de petite taille grâce auxquels le gène xylE peut s'exprimer dans E. coli.

Dans un deuxième temps, à partir de ces "sources" de gène xylE, on va construire des plasmides grâce auxquels le gène xylE pourra s'exprimer dans B. subtilis.

Cette construction est effectuée en deux étapes, tout d'abord le clonage du gène xylE sur un plasmide capable de se répliquer dans B. subtilis, puis la sélection d'un promoteur assurant l'expression du gène xylE dans B. subtilis.

EXEMPLE 2 - Clonage et expression du gène codant pour la catéchol 2,3-oxygénase dans Bacillus subtilis

2a) Souches et plasmides

Les souches de Bacillus subtilis qui ont été utilisées sont dérivées de la souche Marburg 168.

Une souche BZ2 cysB3 recE4 a été construite en transformant à l'aide d'un ADN donneur BD224 thr5 trpC2 recE4 dans une souche KS27 hisA1 cysB3

La sélection a été effectuée pour des colonies $His^+$ sensibles à la mitomycine C (MMC) à une concentration finale de 0,10 µg/ml. La sensibilité à des niveaux faibles de MMC constitue une indication d'une déficience au niveau de la recombinaison associée avec la présence d'une mutation recE4 (réf. 4, 5).

La souche TGB1 trpC2 recE4 spo331 a été obtenue par transformation de cellules réceptrices RUB331 thyA1 thyB1 trpC2 spo331 à l'aide d'un ADN donneur dérivé de BD224. La souche MI112 (leuB8 arg15 thr5 recE4 $r_M^-$ $m_M^-$(Tanaka 197) )a été également utilisée. La sélection a été effectuée pour des cellules $Thy^+$ sensibles à la MMC.

Les souches d'E. coli utilisées sont dérivées de BZ18 $rk^-$ $mk^+$ gal lacY SuII et de C600 $rk^-$ $mk^+$ et CSR603 (réf. 19).

Le plasmide pTG206 a déjà été mentionné dans l'exemple 1.

Le plasmide pHV33, dont la carte de restriction est représentée figure 2B, est décrit dans les références 16 et 18. Ce plasmide est capable de se répliquer dans E. coli en rendant les cellules hôtes résistantes à l'ampicilline (50 µg/ml) $Ap^R$, à la tétracycline (15 µg/ml) $Tc^R$ et au chloramphénicol (20 µg/ml) $Cm^R$. Ce plasmide se réplique également dans B. subtilis ; toutefois, dans ce dernier hôte la résistance au chloramphénicol (5 µg/ml) est la seule à s'exprimer. La partie en trait plus épais de pHV33 sur la figure 2B représente la partie utilisée dans la construction de pTG402 (voir exemples suivants).

2b) Milieu de croissance et conditions de culture

Toutes les souches de B. subtilis ont été maintenues sur un milieu gélose-sang-tryptose (TBAB, Difco Laboratories) auquel on ajoute 0,3 % (poids/volume) de gélose, de thymine et d'uracile, chacun à une concentration finale de 20 µg/ml. Du chloramphénicol (Sigma Chemical Corporation) est ajouté au TBAB à 5 µg/ml pour maintenir les plasmides dans les souches.

La croissance des cultures cellulaires dans des milieux liquides riches est effectuée dans un bouillon de Penassay (PAB, Difco Laboratories) auquel on ajoute 20 µg/ml de thymine. Du chloramphénicol est ajouté lorsque cela est nécessaire.

Toutes les souches d'Escherichia coli sont maintenues sur L-agar. Les cultures liquides sont effectuées en bouillon L. Des antibiotiques sont ajoutés lorsque cela est nécessaire.

Toutes les cultures sont effectuées à 37°C et l'aération desdites cultures est effectuée par agitation.

Les cultures de Bacillus subtilis, une fois qu'elles sont établies, sont maintenues sur TBAB à la température de la pièce (20-22°C). Les cultures d'E. coli sont maintenues sur L-agar à 4°C.

2c) Transformation bactérienne

La transformation des cellules réceptrices d'E. coli par un ADN de plasmide est effectuée par la méthode de Lederberg et Cohen (réf. 10) tel que cela est décrit dans l'article de Morrison (réf. 13). Les cellules compétentes sont préservées par congélation avant leur transformation.

La transformation conventionnelle de B. subtilis (réf. 20) est suivie par la mise en oeuvre de la méthode de Boylan (réf. 2). Il est également possible d'effectuer la transformation des protoplastes en utilisant les ADN plasmidiques (réf. 3). La sélection des souches portant les plasmides est effectuée sur milieu TBAB supplémenté avec 5 µg/ml de chloramphénicol dans le cas de transformations conventionnelles et sur un milieu de régénération DM(3) supplémenté avec 10 µg/ml de chloramphénicol dans le cas d'une transformation de protoplastes.

L'expression fonctionnelle du gène codant pour la C 2,3-O est observée et mesurée comme cela a été décrit à l'exemple 1.

2d) Construction du plasmide pTG402

Le plasmide pHV33 est mis à digérer avec l'endonucléase de restriction BamH1 dans une solution comportant 6mM tris (hydroxyméthyle) aminométhane (Tris) pH 7,9, 6mM $MgCl_2$, 100mM NaCl, 1 µg ADN et 6 unités d'enzyme.

La réaction est conduite pendant 1 heure à 37°C avant d'être stoppée par chauffage pendant 10 minutes à 65°C. Les conditions de tampon sont ajustées à l'aide de 100 mM Tris, pH 7,4, 5mM $MgCl_2$ et 50mM NaCl, puis l'on ajoute 3 unités d'endonucléase de restriction EcoR1.

L'incubation est poursuivie pendant 1 heure à 37°C. La réaction est de nouveau stoppée par chauffage.

Le plasmide pTG206 est traité de la même façon que le plasmide pHV33.

0,5 µg de chaque fragment plasmidique sont ligaturés dans 50 µl de tampon qui contient 66 mM tris HCl, pH 7,9, 6,6 mM $MgCl_2$, 10 mM dithiothréitol (DTT), 0,5 mM adénosine triphosphate (ATP) et 0,1 unité

ADN ligase T4 (Boehringer Mannheim). La réaction est effectuée à 10-12°C pendant approximativement 18 heures, puis stoppée par chauffage de la solution pendant 10 minutes à 65°C.

Les produits obtenus sont stockés à 4°C ou bien sont utilisés directement pour la transformation.

Ces produits de ligation sont utilisés pour transformer E. coli BZ18. Les souches réceptrices sont sélectionnées pour leur résistance à l'ampicilline et au chloramphénicol et leur sensibilité à la tétracycline.

On obtient ainsi 7,6 x $10^4$ transformants par µg ADN plasmidique.

Ces transformants sont pulvérisés avec une solution de catéchol et les colonies qui virent au jaune sont transférées sur des boîtes de sélection contenant de l'ampicilline.

Parmi ces colonies chez lesquelles le gène xylE s'exprime fonctionnellement, 14 sont prélevées et mises en culture 1 ml de culture et l'ADN plasmidique en est extrait puis résolu sur gel d'agarose.

La majorité des plasmides extraits sont plus grands que pTG206 et présentent un site de restriction BamHl et 4 sites de restriction ClaI.

L'un de ces plasmides appelé pTG402 a été purifié à partir d'un litre de culture et analysé avec diverses enzymes de restriction.

Ce nouveau plasmide pTG402 porte le gène C 2,3-O et est capable de se répliquer à la fois dans E. coli et dans B. subtilis. Dans ce dernier hôte toutefois, le produit du gène C 2,3-O codé par pTG402 n'est pas exprimé fonctionnellement, en effet tous les transformants de

*Bacillus subtilis* TGB1 résistant au chloramphénicol demeurent blancs lorsqu'on les pulvérise avec du catéchol. Ceci suggère que le gène C 2,3-O n'a pas été transcrit ou traduit dans *B. subtilis*, ou bien a été réarrangé dans les processus de transformation ou de croissance.

Le plasmide a été extrait de *B. subtilis* pour être ensuite réintroduit dans *E. coli*. Toutes les souches d'*E. coli* transformées deviennent jaunes lorsqu'on les pulvérise avec du catéchol.

On démontre que le gène C 2,3-O est demeuré intact sur le plasmide vecteur dans *B. subtilis* mais qu'il ne s'exprime pas. Ainsi, on peut conclure à une absence d'expression du gène C 2,3-O dans les souches gram + .

2e) Analyse par restriction de pTG402

En utilisant diverses enzymes de restriction pour digérer pTG402, on obtient la carte de restriction partielle représentée figure 2C. La zone en trait épais représente *xylE* et la flèche indique le sens de transcription du mARN. Le tableau 3 rassemble cette analyse de restriction de pTG402.

TABLEAU 3

ANALYSE DES SITES D'ENZYMES DE RESTRICTION DE pTG402

| Enzyme | Nombre total de sites | Nombre de sites dans la région BamHI à XhoI/SalI |
|---|---|---|
| AvaI | 2 | 1 |
| BamHI | 1 | 1 |
| BglII | 0 | 0 |
| ClaI | 4 | 1 |
| EcoRI | 1 | 0 |
| HindIII | 2 | 0 |
| HpaI | 1 | 1 |
| KpnI | 1 | 1 |
| PstI | 1 | 0 |
| SacI | 0 | 0 |
| SalI | 3 | 3 |
| SmaI | 0 | 0 |
| SphI | 0 | 0 |
| XbaI | 0 | 0 |
| XhoI | 0 | 0 |
| XmaI | 0 | 0 |

Ce plasmide de 9,0 kb est constitué d'ADN dérivé de pBR322 (moins la petite région située entre BamHI et SalI), de pC194 et du fragment BamHI à XhoI de pWWO qui contient le gène C 2,3-O.

pBR322 et pC194 ont déjà été séquencés(réf.21 et 35). La région de pTG402 qui contient le gène C 2,3-O a été partiellement séquencée. Cette région est insérée comme un fragment BamHI-XhoI/SalI dans le plasmide pHV33.

Dans ce fragment il existe des sites uniques pour HpaI, AvaI, ClaI et KpnI et 3 sites pour SalI. Il n'existe pas de site BglII, EcoRI, HindIII, SacI, SmaI, SphI, XbaI, XhoI ou XmaI. Les sites HpaI et KpnI sont uniques pour l'ensemble du plasmide pTG402. Le site AvaI se situe à l'intérieur du gène C 2,3-O car lorsque l'on découpe pTG402 avec AvaI et lorsque l'on effectue le ligature des fragments résultants dans une orientation opposée à l'orientation d'origine, ceci supprime l'activité du gène.

2f) Sélection des promoteurs permettant la transcription du gène C 2,3-O dans Bacillus subtilis

L'ADN chromosomique est isolé de la souche de Bacillus subtilis BZ2. L'ADN obtenu est mis à digérer avec l'endonucléase de restriction Sau3A dans un tampon constitué de 6 mM tris, HCl, pH 7,4, 6 mM $MgCl_2$, 50 mM NaCl et 2 à 3 unités d'enzyme Sau3A pour 1 µg d'ADN. On obtient ainsi plusieurs centaines de petits fragments d'ADN avec des extrémités 5' GATC 3' à monobrin cohésives. Ces fragments sont ligaturés avec pTG402 qui a été linéarisé à l'aide de BamHI. (Cette linéarisation produit des terminaisons monobrin identiques à celles produites par Sau3A.)

Les produits de ligature sont introduits directement dans les protoplastes de Bacillus subtilis TGB1 par transformation. D'autre part, les produits de ligature sont introduits par transformation dans Escherichia coli C600 $rk^{-}$ $mk^{+}$.

Les transformants d'E. coli sont sélectionnés par incubation pendant une nuit dans un bouillon L supplémenté avec 50 µg/ml d'ampicilline. La population cellulaire mixte est traitée par la méthode des lysats clarifiés (réf. 7, 12) de façon à isoler les ADN plasmidiques. Une petite proportion d'ADN ainsi obtenu est utilisée comme ADN donneur pour la transformation de B. subtilis TGB1 ou MI112, et ce par les méthodes classiques de transformation. La sélection des cellules comportant un plasmide est effectuée pour la résistance au chloramphénicol.

Tous les transformants TGB1 ou MI112 qu'ils soient obtenus par les méthodes classiques ou bien par transformation protoplastique, sont soumis à une pulvérisation d'une solution de catéchol de façon à détecter l'expression du gène C 2,3-O. La coloration jaune des colonies est utilisée comme une indication pour sélectionner les promoteurs qui permettent la transcription du gène C 2,3-O.

La transformation directe dans les protoplastes a conduit à isoler sept colonies qui deviennent jaunes lorsqu'on les pulvérise avec du catéchol. Les ADN plasmidiques qui se trouvent à l'intérieur de ces cellules ont été appelés pTG411, pTG423, pTG424, pTG425, pTG426, pTG427 et pTG428.

L'utilisation d'*Escherichia coli* comme intermédiaire a permis d'isoler 18 colonies supplémentaires de *B. subtilis* qui deviennent jaunes lorsqu'on les pulvérise avec du catéchol. Les plasmides ainsi obtenus, nommés pTG403, pTG404, pTG405, pTG406, pTG407, pTG408, pTG409, pTG410, pTG412, pTG413, pTG414, pTG415, pTG416, pTG417, pTG418, pTG419, pTG420 et pTG421, demeurent associés avec une activité du gène C 2,3-O, sous réserve que la sélection pour la résistance au chloramphénicol soit maintenue. Les origines de ces différents plasmides sont rassemblées dans le tableau 4.

TABLEAU 4

SELECTION DES PROMOTEURS DANS BACILLUS SUBTILIS

| Expérience | ADN donneur | Méthode de transformation | Plasmide |
|---|---|---|---|
| 1 | BZ2 + pTG402 (3:1) ligature | B. subtilis TGB1 protoplaste | pTG411 |
| 2 | BZ2 + pTG402 (3:1) ligature | E. coli BZ18 | |
| 3 | lysats clarifiés de exp. 2 | B. subtilis TGB1 classique | pTG403 |
| 4 | BZ2 + pTG402 (4:1) ligature | E. coli BZ18 | |
| 5 | lysats clarifiés de exp. 4 | B. subtilis TGB1 classique | pTG404<br>pTG412<br>pTG413 |
| 6 | BZ2 + pTG402 (2:1) ligature | E. coli C600 $rk^{-}$ $mk^{+}$ | |
| 7 | lysats clarifiés de exp. 6 | B. subtilis MI112 classique | pTG405<br>pTG406<br>pTG407<br>pTG408<br>pTG409<br>pTG410<br>pTG414<br>PTG415<br>pTG416<br>pTG417<br>pTG418<br>pTG419<br>pTG420<br>pTG421 |
| 8 | BZ2 + pTG402 (2:1) ligature | B. subtilis MI112 protoplaste | pTG422<br>pTG423<br>pTG424<br>pTG425<br>pTG426<br>pTG427 |

2g) Purification des ADN plasmidiques, digestion par les enzymes de restriction et électrophorèse.

Les ADN plasmidiques sont isolés à partir des lysats d'un bouillon de culture de 1 litre dans un milieu liquide riche.

La purification des ADN plasmidiques est effectuée par centrifugation sur les gradients de densité au chlorure de césium-bromure d'éthidium (réf. 17). Dans certains cas, les ADN plasmidiques sont préparés à partir de culture de 1 ml par la méthode d'extraction alcaline (réf. 1).

Le plasmide pTG402 purifié est mis à digérer avec différentes enzymes de restriction dans les conditions prévues pour leur utilisation. Les dérivés de pTG402 susceptibles de promouvoir l'expression du gène C 2,3-O sont mis à digérer avec BamH1 de façon à rechercher des sites potentiels en aval du promoteur mais en amont du gène C 2,3-O.

Ces fragments d'ADN sont résolus sur gel d'agarose 1 % (poids/volume) en utilisant un appareil d'électrophorèse horizontal. On utilise pour ce faire un tampon Tris-acétate (40 mM Tris, pH 7,8, 20 mM acétate de sodium, 2 mM EDTA).

L'électrophorèse est effectuée sous différence de potentiel constante de 35 volts pendant 14 à 18 heures ou 100 volts pendant 2 à 3 heures à la température de la pièce (20-22°C).

L'ADN est "coloré" grâce au bromure d'éthidium 0,5 µg/ml pendant 30 minutes, le gel est rincé abondamment avec de l'eau distillée, les bandes d'ADN sont visualisées avec des rayons ultra-violets de grandes longueurs d'ondes provenant d'un transilluminateur UV (UltraViolet Products, Inc.). La dimension des fragments d'ADN plasmidiques

est estimée en comparant la mobilité relative à celle de l'ADN de λCI857S7 mis à digérer avec HindIII. La taille des fragments d'ADN est obtenue à partir de la publication de Philippsen et al. (réf. 15).

Chacun des plasmides testés est plus grand que pTG402.

Ce résultat démontre que le gène C 2,3-O est exprimé fonctionnellement dans *B. subtilis*, à condition d'introduire des séquences d'ADN chromosomiques de *B. subtilis* qui assurent la promotion de la transcription du gène C 2,3-O.

2h) Activité enzymatique C 2,3-O dans *Bacillus subtilis*

Des extraits de cellules provenant de culture en phase stationnaire ont été préparés pour déterminer le niveau d'activité enzymatique C 2,3-O dans *B. subtilis*. Ces activités sont mesurées par la méthode spectrophotométrique décrite précédemment et rassemblées dans le tableau 5.

TABLEAU 5

ACTIVITE DE LA CATECHOL 2,3-OXYGENASE DANS BACILLUS SUBTILIS

| Souche et plasmide | N° déterminations | Protéine totale (a) (mg/ml) | Activité (b) (mU/mg) |
|---|---|---|---|
| TGB1/pTG402 | 2 | 1,59 | 0 |
| TGB1/pTG403 | 2 | 1,59 | 238,0 |
| TGB1/pTG404 | 2 | 1,59 | 20,5 |
| MI112/pTG405 | 2 | 1,86 | 1 702,5 |
| MI112/pTG406 | 3 | 1,74 | 124,0 |
| MI112/pTG407 | 2 | 2,22 | 373,5 |
| MI112/pTG408 | 2 | 2,21 | 1 175,0 |
| MI112/pTG409 | 2 | 2,03 | 169,5 |
| MI112/pTG410 | 2 | 1,93 | 961,5 |
| TGB1/pTG411 | 3 | 1,02 | 58,0 |
| TGB1/pTG412 | 2 | 1,30 | 24,0 |
| TGB1/pTG413 | 2 | 1,60 | 14,0 |
| MI112/pTG414 | 2 | 1,54 | 288,0 |
| MI112/pTG415 | 2 | 1,90 | 696,0 |
| MI112/pTG416 | 2 | 2,30 | 2 011,0 |
| MI112/pTG417 | 2 | 1,93 | 1 853,0 |
| MI112/pTG418 | 2 | 1,92 | 217,0 |
| MI112/pTG419 | 2 | 2,15 | 487,0 |
| MI112/pTG420 | 3 | 1,90 | 551,0 |
| MI112/pTG421 | 2 | 1,54 | 245,0 |
| MI112/pTG422 | 2 | 2,23 | 192,0 |
| MI112/pTG423 | 2 | 1,73 | 278,0 |
| MI112/pTG424 | 2 | 2,47 | 68,0 |
| MI112/pTG425 | 2 | 1,93 | 79,0 |
| MI112/pTG426 | 2 | 1,80 | 93,0 |
| MI112/pTG427 | 2 | 1,89 | 113,0 |

(a) Concentrations en protéines par la méthode de Lowry

(b) 1 milliunité (mU) correspond à la formation de 1 mmol. de semialdéhyde de 2-hydroxymuconique par minute à 30°C

Remarque: Il a été observé que l'activité de la C 2,3-O dans des extraits de B. subtilis peut être augmentée par dialyse de l'extrait contre un tampon contenant du $Fe^{2+}$.

21) Caractérisation des plasmides de Bacillus subtilis qui expriment le gène C 2,3-O

La ligation d'ADN généré par Sau3A à un plasmide vecteur linéarisé par BamH1 (tel que pTG402) prévoit avec une probabilité de 1/4 la régénération d'un site BamH1 pour chacune des extrémités du fragment Sau3A. Les plasmides associés avec une activité du gène C 2,3-O dans B. subtilis sont mis à digérer avec BamH1. Les plasmides pTG403, pTG404, pTG405, pTG406, pTG407, pTG408, pTG409, pTG410, pTG411, pTG422 et pTG425 présentent au moins un site BamH1.

Les plasmides purifiés sont mis à digérer par HindIII et BamHI pour déterminer si les sites BamHI régénérés sont en amont ou en aval des promoteurs qui se trouvent localisés sur les fragments Sau3A. Un site BamHI unique est détecté en amont du promoteur sur pTG411, pTG422 et pTG425. Des sites BamH1 uniques sont détectés en aval des promoteurs sur pTG403, pTG404, pTG405, pTG406, pTG407, pTG408, pTG409 et pTG410.

Les dimensions approximatives du fragment Sau3A qui porte les promoteurs de Bacillus subtilis sont de 500 paires de base pour pTG403, 140 paires de base pour pTG404, 575 paires de base pour pTG405, 950 paires de base pour pTG406, 335 paires de base pour pTG407, 500 paires de base pour pTG408, 900 paires de base pour pTG409 et 500 paires de base pour pTG410.

2j) Développement des vecteurs d'expression plasmidiques dans Bacillus subtilis

Les plasmides pTG403, pTG404, pTG405, pTG406, pTG407, pTG408, pTG409 et pTG410 présentent les caractéristiques suivantes :

1°) Ils expriment le gène C 2,3-O.

2°) Les cellules hôtes qui ont reçu ces plasmides convertissent le catéchol en semialdéhyde 2-hydroxymuconique, comme cela peut être mis en évidence par coloration en jaune des colonies et analyse spectrophotométrique.

3°) Ils présentent un site unique BamH1 en aval du promoteur mais en amont du gène C 2,3-O.

Ces plasmides sont importants, non seulement pour la conversion du catéchol, mais aussi parce qu'ils peuvent être également utilisés comme vecteurs d'expression dans Bacillus subtilis.

Le clonage d'un gène dans le site BamH1, aussi bien de pTG403, pTG404, pTG405, pTG406, pTG407, pTG408, pTG409 et pTG410, aura selon toute probabilité pour effet de supprimer l'activité du gène C 2,3-O. De même, le clonage de gène dans la région de pTG403, pTG404, pTG405, pTG406, pTG407, pTG408, pTG409 et pTG410, qui se situe entre le site BamHI et un site quelconque à l'intérieur du gène C 2,3-O, aura probablement pour effet de supprimer l'activité du gène C 2,3-O.

Ainsi, l'insertion de nouveaux gènes dans le vecteur d'expression peut être détectée en pulvérisant les colonies avec une solution de catéchol et en observant l'apparition ou non d'une coloration jaune. En effet, les colonies qui demeurent blanches indiquent que le gène C 2,3-O n'est plus exprimé et qu'un nouveau gène a été inséré dans le plasmide. Par contre, les

colonies jaunes indiquent que ce nouveau gène n'a pas été inséré dans le plasmide. Un nouveau gène inséré dans le site BamHI a une grande probabilité d'être exprimé parce qu'un promoteur de *B. subtilis* est situé en amont de ce site BamHI.

Dans ces conditions, les plasmides ainsi obtenus présentent un très grand intérêt sur le plan industriel pour l'expression d'un gène déterminé dans *Bacillus subtilis*.

EXEMPLE 3 - Clonage et expression du gène dans le phage M13

Le phage M13 est un coliphage filamenteux distribué par Bethesda Research Laboratories et PL Labs. sous forme de "kit" pour le séquençage et le clonage de gènes.

Le fragment BamHI-XhoI de pTG200 a été cloné dans les sites BamHI-SalI du phage M13.

Le phage ainsi obtenu permet l'expression du gène xylE dans les souches d'*Escherichia coli* mentionnées précédemment.

Le clonage de ce gène est mis en évidence, comme cela a été indiqué à l'exemple 1, par pulvérisation de catéchol et observation des plages de lyse de couleur jaune dû à la dégradation du catéchol.

Les deux fragments SalI (voir figure 1) contenus dans le segment BamHI-XhoI ont été clonés dans M13 de deux façons :

- en conservant leur sens et leur ordre originaux,
- en conservant leur ordre original mais en inversant leur sens.

Seul le phage recombinant construit selon le premier mode a pu exprimer une activité de C 2,3-O.

EXEMPLE 4 - Sélection de fragments promoteurs de l'expression du gène xylE dans Bacillus subtilis

Afin de déterminer si des fragments d'ADN provenant :

- du chromosome d'une espèce bactérienne autre que B. subtilis,
- d'un plasmide bactérien, ou
- d'un bactériophage,

pouvaient également être des promoteurs de l'expression de xylE dans B. subtilis, des ADN provenant de diverses sources ont été isolés puis purifiés.

Ces sources d'ADN comportent notamment :

- Bacillus licheniformis 9945A,
- Bacillus pumilus BP1,
- Escherichia coli C600 $rk^- mk^+$,
- le plasmide pUB110 (isolé à l'origine de Staphylococcus aureus et dont il a été démontré par la suite qu'il se répliquait dans B. subtilis (référence 30), et
- le bactériophage $\phi$29 de B. subtilis (plus particulièrement $\phi$29susS14 (1242) (référence 31).

Dans le cas de l'ADN chromosomique de B. licheniformis, B. pumilus et E. coli, et de l'ADN provenant de $\phi$29, les ADN après purification sont mis à digérer avec Sau3A.

Les fragments ainsi obtenus sont mis en ligation avec le plasmide pTG402 qui a été au préalable mis en digestion avec BamHI. Des rapports en masse 2:1 (ADN insérés:pTG402) ont été utilisés dans ce cas.

Pour ce qui concerne le plasmide pUB110 utilisé comme source d'ADN à insérer, pUB110 a été mis en digestion avec BamHI et BglII. Ceci conduit à deux fragments d'ADN, l'un ayant approximativement 1,55 kb

et l'autre ayant approximativement 3,95 kb. Ces résultats sont en accord avec la carte de restriction connue de ce plasmide commercialisé, notamment, par Bethesda Research Laboratories.

Les fragments d'ADN de pUB110 sont mis en ligation avec pTG402 après digestion de ce dernier par BamHI dans un rapport égal en poids.

Les produits de ligation de chacune de ces expériences sont introduits séparément dans B. subtilis MI112 par transformation de protoplastes.

Les transformants $Cm^r$ sont sélectionnés après 48 heures d'incubation à 37°C sur un milieu de régénération DM3 supplémenté avec 10 µg/ml de Cm.

Les colonies de transformants $Cm^r$ sont pulvérisées avec du catéchol et des colonies jaunes sont observées dans tous les cas.

Ceci démontre que le gène xylE de pTG402 peut être exprimé dans B. subtilis sous le contrôle d'ADN isolés d'autres espèces de Bacillus.

Il en va de même pour les ADN extraits du microorganisme E. coli gram -, du plasmide pUB110 et du bactériophage ϕ29.

Lorsque l'ADN de B. licheniformis est inséré dans pTG402 les colonies jaunes sont observées à une fréquence de $5,68.10^{-3}$ du total des transformants $Cm^r$.

Pour l'ADN de B. pumilus, une fréquence de $1,93.10^{-3}$ colonies jaunes par transformant $Cm^r$ est observée.

Lorsque l'ADN donneur est celui de E. coli, on obtient seulement deux colonies jaunes pour plus de $10^4$ transformants $Cm^r$ (fréquence inférieure ou égale à $2.10^{-4}$).

Ces derniers résultats sont tout à fait inattendus compte tenu des barrières régissant les expressions de gènes hétérospécifiques dans B. subtilis, (réf. 32), mais ceci démontre que le gène xylE de Pseudomonas peut être exprimé dans B. subtilis sous le contrôle d'un ADN provenant de E. coli.

Les activités spécifiques en C 2,3-O produites dans ces expériences et la taille approximatives des inserts d'ADN sont représentées dans le tableau 6.

TABLEAU 6

| Plasmide | Source d'insert | Taille de l'insert (bp) | Activité spécifique en C 2,3-O (mU/mg) |
|---|---|---|---|
| pTG431 | pUB110 | 1 550 | 1 473,0 |
| pTG432 | *E. coli* | 600 | 42,0 |
| pTG433 | " | 500 | 324,0 |
| pTG435 | *B. pumilus* | 200 | 1 794,0 |
| pTG436 | " | 550 | 327,0 |
| pTG437 | " | 1 850 | 725,0 |
| pTG438 | *B. licheniformis* | 700 | 555,0 |
| pTG439 | " | 400 | 2 240,0 |
| pTG440 | " | 600 | 1 018,0 |
| pTG441 | " | 500 | 2 727,0 |
| pTG442 | ϕ29 | 300 | 1 853,0 |
| pTGϕ29p1 | " | non testé | 262,7 |
| pTGϕ29p2 | " | " | 215,4 |
| pTGϕ29p3 | " | " | 928,9 |
| pTGϕ29p4 | " | " | 1 136,0 |
| pTGϕ29p5 | " | " | 29,2 |
| pTGϕ29p6 | " | " | 1 295,0 |
| pTGϕ29p7 | " | " | 1 100,0 |
| pTGϕ29p9 | " | " | 27,3 |
| pTGϕ29p11 | " | " | 460,0 |
| pTGϕ29p12 | " | " | 764,0 |
| pTGϕ29p13 | " | " | 769,0 |
| pTGϕ29p14 | " | " | 455,0 |
| pTGϕ29p15 | " | " | 261,0 |
| pTGϕ29p16 | " | " | 1 017,0 |
| pTGϕ29p17 | " | " | 993,0 |
| pTGϕ29p18 | " | " | 869,0 |
| pTGϕ29p19 | " | " | 1 111,0 |
| pTGϕ29p20 | " | " | 789,0 |

Les activités spécifiques les plus importantes pour C 2,3-O sont obtenues avec MI112/pTG441 dans lequel l'insert d'ADN provient de B. licheniformis 9945A.

Bien que 20 plasmides exprimant xylE sous le contrôle de $\phi$29 soient isolés, certains d'entre eux semblent être identiques.

EXEMPLE 5 - Séquence des nucléotides du gène xylE et site de fixation des ribosomes

La figure 3 représente la séquence de nucléotides complète du gène xylE et la séquence d'aminoacides correspondante.

La séquence de nucléotides commence à 100 paires de bases en aval du site HpaII le plus proche de l'extrémité 5' et se termine au site HpaII à l'extrême droite (extrémité 3') (voir aussi fig. 4).

Le site probable de fixation des ribosomes est souligné.

La région codante de xylE commence à la base 1 et se termine au codon souligné par trois astérisques (fig. 3) ; elle est représentée par des flèches verticales dans la figure 4.

La stratégie utilisée pour le sous-clonage et le séquençage des fragments du gène xylE dans le bactériophage M13 est représentée par des flèches horizontales à la figure 4.

La localisation du site d'initiation de la traduction et le schéma de lecture représentés sont en parfait accord avec la séquence des 9 premiers aminoacides déterminés à partir de C 2,3-O purifiée.

Le gène code pour 306 aminoacides qui constituent un polypeptide de 35 047 daltons.

Comme la forme active de C 2,3-O présente un poids moléculaire de 140 000 daltons (référence 33), ceci confirme que la C 2,3-O est constituée par 4 sous-unités identiques (référence 34).

Une complémentation importante entre le site de fixation des ribosomes et la région 3' du rARN 16S semble être nécessaire pour l'initiation de la traduction dans *B. subtilis*.

Le site de fixation des ribosomes proposé pour le gène *xylE* permet de prédire que ΔG, l'énergie libre d'appariement des bases entre ce site et le rARN 16S de *B. subtilis*, est de - 15,6 kilocalories. En plus, la distance entre le site de fixation des ribosomes et le codon d'initiation est de 9 bases.
Les deux critères tombent dans les ordres de grandeur de ΔG et le nombre de bases d'espacement récemment calculé pour une série de sites de fixation de ribosomes dans le cas de *B. subtilis*. (référence 36)

Ces observations confirment que l'initiation de la traduction de *xylE* mARN dans *B. subtilis* démarre au même codon que celui qui est reconnu dans *Pseudomonas* et *E. coli*.

EXEMPLE 6 - Etude de l'identité de C 2,3-O dans *P. putida, E. coli* et *B. subtilis*

Afin de démontrer que l'enzyme produite par *B. subtilis* est identique à celle produite par *P. putida* mt-2 ou *E. coli* BZ18/pTG206, les extraits acellulaires partiellement purifiés de chacun des organismes sont résolus par électrophorèse sur gel de polyacrylamide; les résultats obtenus sont représentés sur la figure 5.

L'électrophorèse sur gel de polyacrylamide de C 2,3-O est effectuée de la façon suivante :

Des préparations de C 2,3-O partiellement purifiées sont obtenues à partir de cultures récoltées en phase stationnaire (réf. 9). Les cellules sont lavées avec un tampon phosphate (20 mM), pH 7,2, et remises en suspension dans un tampon AP (acétone 10 %, phosphate 100 mM, pH 7,5) puis traitées aux ultrasons.

Les débris cellulaires sont éliminés par centrifugation à 30 000 rpm pendant 1 heure à 4°C dans une ultracentrifugeuse L8-70 avec un rotor Beckman R65Ti. Le surnageant est traité à l'acétone jusqu'à une concentration finale de 50 % v/v et les protéines précipitées à 4°C pendant 2 heures.

Une nouvelle centrifugation à 10 000 rpm pendant 10 minutes dans une centrifugeuse RC-5B avec un rotor HB-4 produit un surnageant qui est amené à 66 % v/v d'acétone et les protéines sont précipitées pendant 2 heures à 4°C.

Après centrifugation pendant 10 minutes à 10 000 rpm, les culots de protéines sont remis en suspension dans 1 ml de tampon AP.

Les activités enzymatiques sont mesurées comme cela a été décrit précédemment.

Pour l'électrophorèse, on utilise un gel de séparation à 7 % d'acrylamide et un gel de support à 3 %.

20 à 200 µg d'extraits sont déposés sur gel de manière à ce que l'activité C 2,3-O soit la même dans chaque logette (soit 260 mU). Les échantillons analysés correspondent de gauche à droite, à des extraits de P. putida mt-2, E. coli BZ18/pTG206, B. subtilis TGB1/pTG402, B. subtilis TGB1/pTG403.

Pour TGB1/pTG402, une quantité de protéines équivalente à celle de TGB1/pTG403 est utilisée. L'électrophorèse est effectuée sous 25 milliampères, à courant constant pendant 5 heures.

La plaque A est pulvérisée avec du catéchol 0,5 M. Sur la plaque B, les protéines sont fixées puis colorées avec le bleu brillant Coomassie R250 (0,15 % poids/volume) puis décolorées dans une solution de 7,5 % v/v d'acide acétique et 5 % v/v de méthanol. La position de la catéchol oxygénase est indiquée par une flèche.

Comme l'électrophorèse est réalisée en conditions non dénaturantes, l'enzyme C 2,3-O est identifiée aisément en pulvérisant le gel avec du catéchol.

Des bandes jaunes apparaissent à la même distance de migration pour tous les extraits, à l'exception de celui de la souche B. subtilis (TGB1/pTG402) qui porte xylE sans le promoteur fonctionnel. Pour ce dernier il n'est pas observé de bande jaune.

L'identité des mobilités électrophorétiques de C 2,3-O suggère que l'enzyme dans B. subtilis est produite sous forme de polypeptide fusionné mais qu'il présente le même produit de traduction que celui observé dans Pseudomonas et E. coli.

Dans une expérience supplémentaire, un antisérum brut,obtenu à partir de lapins auxquels on a injecté la C 2,3-O purifiée de E. coli, est testé selon la méthode de Outchterlony en double diffusion. Ce test immunologique démontre que l'antisérum réagit avec les extraits partiellement purifiés de P. putida mt-2, E. coli BZ18/pTG206 et B. subtilis TGB1/pTG403, mais n'a pas de réaction croisée avec l'extrait de B. subtilis préparé à partir de TGB1/pTG402.

EXEMPLE 7

Cet exemple a pour objet de démontrer l'activité de décontamination de la catéchol 2,3-oxygénase vis-à-vis de l'urushiol.

71. Produits

- Urushiol : solutions 0,1 % ou 1 % dans l'éthanol.
- Catéchol oxygénase : enzyme purifiée à 90 % contenant 1 000 unités/ml provenant soit de E. coli (souche BZ18/pTG203) ou de B. subtilis (souche MI112/pTG401). Il a été démontré que l'enzyme est identique quelle que soit la souche hôte utilisée.
- Lessives commerciales :
  1) WIPP (marque déposée commercialisée par S.A. Henkel Belgium N.V.) utilisée à une concentration finale de 0,25 % dans l'eau de ville,
  2) DASH (marque déposée commercialisée par Procter & Gamble, Benelux) utilisée à une concentration finale de 0,36 % dans l'eau de ville.

  WIPP est une lessive "à la main" dont le pH est de 6,8 environ ;
  DASH est une poudre destinée à la lessive en machine dont le pH est égal à 9.
  3) Différentes solutions détergentes ont été élaborées en laboratoires. Elles sont constituées par 10 mM de phosphate de sodium, pH 6,8 + soit Brij 58 (polyoxyéthylène-20-cétylester) 0,025 %, ou SDS (dodécyl sulfate de sodium) 0,02 %.
  4) Bain mousse PALMOLIVE qui est utilisé à une concentration finale de 0,01 %.
- Tissus : 5 types différents dont les 4 premiers sont tissés (tissus de couleur blanche) :

1) coton 100 %
2) coton 35 % + polyester 65 %
3) laine
4) 100 % acrylique
5) 100 % Dacron.

72. Mesure de l'activité de la catéchol 2,3-oxygénase

L'activité enzymatique est mesurée soit par appréciation de la coloration jaune du produit de clivage de l'urushiol par la catéchol 2,3-oxygénase ou par chromatographie en couche mince. Dans cette dernière méthode la quantité d'urushiol restante est mesurée par la taille de la tache correspondante. Ce procédé présente une limite inférieure de détection de 1 µg.

73. Test de l'activité de la catéchol 2,3-oxygénase dans les solutions détergentes commerciales

L'activité de la catéchol 2,3-oxygénase dans les solutions détergentes a été mise en évidence par l'apparition du produit de coloration jaune, le semialdéhyde 2-hydroxymuconique. En utilisant le détergent WIPP on observe une apparition graduelle de la coloration jaune, alors qu'en présence de DASH cette apparition de la coloration jaune est seulement transitoire.

Afin de tester l'activité de la catéchol 2,3-oxygénase sur l'urushiol dans des solutions détergentes, on mélange l'urushiol, l'enzyme et divers détergents puis on laisse le mélange incuber pendant 3 heures à 30°C dans un volume final de 1 ml. Les résultats sont représentés dans le tableau ci-après :

| | Urushiol | Lessive | Acétone 10 % | C 2,3-O | Couleur jaune | Urushiol résiduel (TLC) |
|---|---|---|---|---|---|---|
| 1. | 50 µg | WIPP 0,25% | - | - | - | ≥ 10 µg |
| 2. | 50 µg | WIPP 0,25% | - | 10 u | +++ | < 1 µg |
| 3. | 50 µg | $PO_4$ 10mM SDS 0,01% | - | 10 u | ± | < 1 µg |
| 4. | 10 µg | PALMOLIVE 0,01% | + | - | - | ≥ 5 µg |
| 5. | 10 µg | PALMOLIVE 0,01% | + | 10 u | ++ | < 1 µg |

Le symbole < 1 µg signifie que la quantité d'urushiol restante après incubation est inférieure aux limites de détection de l'analyse.

L'apparition d'une couleur jaune démontre que l'urushiol est effectivement solubilisé par les détergents commerciaux et qu'il est ensuite scindé par la catéchol 2,3-oxygénase.

En présence de l'enzyme pratiquement tout l'urushiol est scindé même lorsqu'on utilise de grandes quantités d'urushiol.

Ce test démontre la stabilité de la catéchol 2,3-oxygénase dans les solutions détergentes et démontre, en outre, que des quantités importantes d'urushiol peuvent être scindées avec des quantités même faibles d'enzyme.

Comme cela apparaît dans le test 1 du tableau précédent, en l'absence de catéchol 2,3-oxygénase l'urushiol est récupéré pratiquement quantitativement. Ceci démontre que la disparition de l'urushiol des solutions détergentes contenant de la catéchol 2,3-oxygénase est due à l'action de l'enzyme.

74. <u>Stabilité de la catéchol 2,3-oxygénase dans une solution détergente</u>

L'étude de la stabilité de la catéchol 2,3-oxygénase en solution dans divers détergents montre qu'après 12 heures

à la température ambiante l'activité enzymatique n'est pas sensiblement diminuée par rapport à une solution témoin qui ne contient pas de détergent.

Toutefois,dans le cas du détergent DASH, on note une réduction de l'activité enzymatique due probablement au pH de la solution.

Après incubation de la catéchol 2,3-oxygénase pendant 15 minutes à 60°C en présence du détergent, plus de 75 % de l'activité enzymatique est maintenue.

Afin d'améliorer la stabilité de l'enzyme, il est intéressant d'incorporer un agent protecteur dans le mélange. A titre d'agent protecteur, l'acétone à une concentration finale de 10 % s'est montrée particulièrement efficace, mais il est possible de la remplacer par d'autres solvants organiques tels que le glycérol par exemple.

75. <u>Activité de la catéchol 2,3-oxygénase sur l'urushiol absorbé sur différents tissus</u>

Les expériences sont conduites de la façon suivante :

10 µl de la solution d'urushiol (0,1 % dans l'éthanol) sont appliqués sur une pièce de tissu de 1 $cm^2$. Le tissu est séché à la température ambiante puis incubé dans 890 µl d'une solution détergente comme indiqué précédemment pendant 3 heures à 30°C (étape de prélavage).

10 unités de catéchol 2,3-oxygénase sont alors ajoutées en même temps que 10 µl d'acétone et l'incubation est poursuivie pendant 3 heures à 30°C.

Après incubation, l'urushiol restant sur les tissus et dans la solution détergente est extrait et dosé. Le tissu est séché et l'urushiol restant est extrait du tissu par 500 µl d'éthanol à la température

ambiante. L'urushiol présent dans la solution détergente est extrait avec 500 µl de butanol.

L'urushiol extrait dans les phases butanolique et éthanolique est récupéré par lyophilisation et remis en suspension dans 25 µl d'éthanol puis appliqué sur une plaque de chromatographie en couche mince.

La chromatographie est effectuée en utilisant de l'éther de pétrole et de l'acétate d'éthyle 7:3 (v/v) à titre de solvant. L'urushiol est identifié après traitement de la plaque avec de l'iode comme cela est décrit par Dupuis (British Journal of Dermatology 101, 617, 1979). Sur chacune des plaques 1,5 et 10 µg d'urushiol sont co-chromatographiés de façon à faciliter la quantification de l'urushiol. A titre de contrôle, on effectue des incubations en l'absence de catéchol 2,3-oxygénase.

Les résultats de ces expérimentations indiquent que bien que l'urushiol soit effectivement éliminé du coton, du coton-polyester, de la laine et des tissus acryliques par simple lavage dans une solution détergente, il n'est pas détruit lorsque la catéchol 2,3-oxygénase est absente.

En présence de catéchol 2,3-oxygénase toutefois, l'urushiol ne peut plus être détecté par chromatographie en couche mince et une coloration jaune indiquant une scission enzymatique du cycle catéchol de l'urushiol apparaît dans la solution de lavage. En l'absence de détergent, l'urushiol demeure absorbé sur les tissus et n'est pas scindé par la catéchol 2,3-oxygénase. Dans les conditions décrites, 10 unités de catéchol 2,3-oxygénase scindent aisément 10 µg d'urushiol, ce qui indique que la quantité d'enzyme utilisée et/ou que le temps d'incubation peuvent être réduits.

76. Conclusion

Les résultats obtenus démontrent que la catéchol 2,3-oxygénase est active et stable en milieu complexe (par exemple solutions détergentes commerciales) et ce dans une fourchette très large de température. La présence d'un agent de stabilisation améliore encore la stabilité de l'enzyme.

La catéchol 2,3-oxygénase élimine de façon efficace l'urushiol lors du lavage de tissus contaminés.

Dépôt de souches

Les souches suivantes ont été déposées à l'Agricultural Research Culture Collection (NRRL) le 14 janvier 1983 :

B15264 : *B. subtilis* TGB1 portant pTG402

B15265 : B. subtilis MI112 portant pTG441.

## BIBLIOGRAPHIE


1. Birnboim H.C. et J. Doly. 1979. A rapid alkaline extraction procedure for screening recombinant plasmid DNA. Nucleic Acids Res. 7: 1513-1520.

2. Boylan R.J., N.H. Mendelson, D. Brooks et F.E. Young. 1972. Regulation of the bacterial cell wall: analysis of a mutant of Bacillus subtilis defective in biosynthesis of teichoic acid. J. Bacteriol. 110: 281-290.

3. Chang S. et S.N. Cohen. 1979. High Frequency transformation of Bacillus subtilis protoplasts by plasmid DNA. Molec. Gen. Genet. 168: 111-115.

4. Dubnau D. et C. Cirigliano. 1974. Genetic characterization of recombination eficient mutants of Bacillus subtilis. J. Bacteriol. 117: 488-493.

5. Dubnau D., R. Davidoff-Abelson, B. Scher et C. Cirigliano. 1973. Fate of transforming deoxyribonucleic acid after uptake by competent Bacillus subtilis. henotypic characterization of radiation sensitive recombination-deficient mutants. J. Bacteriol. 114: 273-286.

6. Franklin F.C.H., M. Bagdasarian, M.M. Bagdasarian et K.N. Timmis. 1981. Molecular and functional analysis of the TOL plasmid pWWO from Pseudomonas putida and cloning of genes for the entire regulated aromatic ring meta cleavage pathway. Proc. Natl. Acad. Sci. U.S.A., in press.

7. Guerry P., D.J. Le Blanc et S. Falkow. 1973. General method for the isolation of plasmid deoxyribonucleic acid. J. Bacteriol. 116: 1064-1066.

8. Hayward G.S. et M.G. Smith. 1972. The chromosome of bacteriophage T5. I. Analysis of the single-stranded DNA fragments by agarose gel electrophoresis. J. Mol. Biol. 63: 383-395.

9. Inouye S., A. Nakazawa et T. Nakazawa. 1981. Molecular cloning of TOL genes *xylB* and *xylE* in *Escherichia coli*. J. Bacteriol. 145: 1137-1143.

10. Lederberg E.M. et S.N. Cohen. 1974. Transformation of *Salmonella typhimurium* by plasmid deoxyribonucleic acid. J. Bacteriol. 119: 1072-1074.

11. Lowry O.H., N.J. Rosebrough, A.L. Farr et R.J. Randall. 1951. Protein measurement with the Folin phenol reagent. J. Biol. Chem. 193: 265-275.

12. Meyers J.A., D. Sanchez, L.P. Elwell et S. Falkow. 1976. Simple agarose electrophoretic method for the identification and characterization of plasmid deoxyribonucleic acid. J. Bacteriol. 127: 1529-1537.

13. Morrison D.A. 1977. Transformation in *Escherichia coli*: cryogenic preservation of competent cells. J. Bacteriol. 132: 349-351.

14. Nakazawa T., S. Inouye et A. Nakazawa. 1980. Physical and functional mapping of RP-4 TOL plasmid recombinants: analysis of insertion and deletion mutants. J. Bacteriol. 144: 222-231.

15. Philippsen P., R.A. Kramer et R.W. Davis. 1978. Cloning of the yeast ribosomal DNA repeat unit in *Sst* I and *Hind* III lambda vectors using genetic and physical size selections. J. Mol. Biol. 123: 371-386.

16. Primrose S.B. et S.D. Ehrlich. 1981. Isolation of plasmid deletion mutants and study of their instability. Plasmid 6: 193-201.

17. Radloff R.W., W. Bauer et J. Vinograd. 1967. A dye-buoyant density method for the detection and isolation of closed circular complex DNA: the closed circular DNA in HeLa cells. Proc. Natl. Acad. Sci. U.S.A. 57: 1514-1521.

18. Rapoport G., A. Klier, A. Billault, F. Fargette et R. Dedonder. 1979. Construction of a colony bank of E. coli containing hybrid plasmids representative of the Bacillus subtilis 168 genome. Molec. Gen. Genet. 176: 239-245.

19. Sancar A., A.M. Hack et W.D. Rupp. 1979. Simple method for identification of plasmid-coded proteins. J. Bacteriol. 137: 692-693.

20. Spizizen J. 1958. Transformation of biochemically deficient strains of Bacillus subtilis by deoxyribonucleate. Proc. Natl. Acad. Sci. U.S.A. 44: 1072-1078.

21. Sutcliffe J.G., 1979. Complete nucleotide sequence of the Escherichia coli plasmid pBR322. Cold Spring Harbor Symp. Quant. Biol. 43: 77-90.

22. Renaut E., P. Stanssens et W. Fiers. 1981. Plasmid vectors for high efficiency expression controlled by the $p_L$ promotor of coliphage lambda. Gene 15: 81-93.

23. Lieb M. 1966. Studies of heat inducible lambda bacteriophage I. order of genetic sites and properties of mutant prophages. J. Mol. Biol. 16: 149-163.

24. Halleweell R.A. et S. Emtage. 1980. Plasmid vectors containing the tryptophan operon promotor suitable for efficient regulated expression of forgein genes. Gene 9: 27-47.

25. Kushner S.R. 1978. An improved method for transformation of Escherichia coli with ColE1 derived plasmids in genetic engineering, Eds Boyer, H.W. et S. Nicosia Elsevier/Morth-Holland Biomedical press. 1978.

26. Murray N.E. et W.J. Brammer. 1973. The tepE gene of Escherichia coli K contains a recognition sequence for the K-restriction system. J. Mol. Biol. 77: 615-624.

27. Franklin F.C.H., M. Bagdasarian et K.N. Timmis. 1981. Manipulation of degregative genes of soil bacteria. Microbiol Degredation of Xenobiotics and Recalatrant Compounds. Eds R. Hutter et T. Leisinger, Academic Press, London 1981.

28. Nozaki M., Kagamiyama H. Hayaishi. Metapyrocatechax. Purification, Crystallization and some Properties. 1963. Biochemische Zeitschrift 338: 582-590.

29. Salat-Trepat José M., Evans C. The meta-cleavage of catechol by Azotobacter species. 1971. Eur. J. Biochem. 20: 400-413.

30. Ehrlick S.D., 1977, Replication and expression of plasmids from Staphylococcus aureus in Bacillus Subtilis. Proc. Natl. Acad. Sci. U.S.A., 74, 1680-1682.

31. Carrascosa J.L., Camacho A., Moreno F., Jimenez F., Mellado R.P., Vinuela E. et Salas M., 1976. Bacillus subtilis phage $\phi$29. Eur. J. Biochem. 66, 229-241.

32. McLaughlin J.R., Murray C.L. et Rabinowitz J.C., 1982. Heterospecific gene expression in Microbiology, 1982, Ed. D. Schlessinger, American Society for Microbiology, Washington D.C., pp. 22-27.

33. Nozaki M. Ono K., Nakazawa T. Kotani S. et Hayaishi O., 1968, Metapyrocatechase II. The role of iron and sulfhydryl groups. J. Biol. Chem. 243, 2682-2690.

34. Nozaki M., 1979, Oxygenases et dioxygenases, Topics Curr. Chem., 78, 145-186.

35. Horinouchi, S. et B. Weishlum, 1982, Nucleotide sequence and functional map of pC194, a plasmid that specifies inducible chloramphenicol resistance. J. Bacteriol. 150, 815-825.

36. Moran C.P. Jr., N. Lang., S.F.J. Le Grice, G. Lee, M. Stephens, A.L. Sonenshein, J. Pero et R. Losick, 1982 ; Nucleotide sequences that signal the initiation of transcription and translation in Bacillus subtilis. Molec. Gen. Genet. 186: 339-346.

REVENDICATIONS

1) Plasmide vecteur caractérisé en ce qu'il comporte au moins :

- le gène xylE codant pour la synthèse de la catéchol 2,3-oxygénase, et

- un promoteur de l'expression fonctionnelle du gène xylE dans une bactérie gram +.

2) Plasmide vecteur selon la revendication 1, caractérisé en ce que la bactérie gram + est un Bacillus, Lactobacillus, Streptococcus, Staphylococcus ou Corynebacterium.

3) Plasmide vecteur selon la revendication 2, caractérisé en ce que la bactérie gram + est un Bacillus subtilis.

4) Plasmide vecteur selon la revendication 1, caractérisé en ce que le promoteur est une séquence d'ADN chromosomique d'une bactérie gram +.

5) Plasmide vecteur selon la revendication 4, caractérisé en ce que l'ADN chromosomique est extrait d'une souche de Bacillus.

6) Plasmide vecteur selon la revendication 5, caractérisé en ce que l'ADN chromosomique est extrait d'une souche de Bacillus chosie parmi : B. subtilis, B. licheniformis, B. pumilus.

7) Plasmide vecteur selon la revendication 1, caractérisé en ce que le promoteur est une séquence d'ADN chromosomique d'une bactérie gram - .

8) Plasmide vecteur selon la revendication 7, caractérisé en ce que le promoteur est une séquence d'ADN chromosomique d'E. coli.

9) Plasmide vecteur selon la revendication 1, caractérisé en ce que le promoteur est une séquence d'ADN d'un plasmide.

10) Plasmide vecteur selon la revendication 9, caractérisé en ce que le promoteur est une séquence d'ADN d'un plasmide se répliquant dans les bactéries gram +.

11) Plasmide vecteur selon la revendication 10, caractérisé en ce que le promoteur est une séquence d'ADN du plasmide pUB110.

12) Plasmide vecteur selon la revendication 1, caractérisé en ce que le promoteur est une séquence d'ADN d'un bactériophage.

13) Plasmide vecteur selon la revendication 12, caractérisé en ce que le promoteur est une séquence d'ADN du bactériophage $\phi$29.

14) Plasmide vecteur selon la revendication 1, caractérisé en ce qu'il est choisi parmi pTG402 à pTG427, pTG431 à pTG442 et pTG$\phi$29p1 à pTG$\phi$29p20.

15) Plasmide vecteur selon la revendication 14, caractérisé en ce qu'il s'agit d'un plasmide dérivé de pTG402.

16) Plasmide vecteur selon l'une des revendications 1 et 2, caractérisé en ce que le plasmide comporte au moins un site unique de restriction situé en un endroit tel que l'insertion d'une séquence d'ADN dans ce site conduise à l'inactivation du gène xylE.

17) Plasmide vecteur selon la revendication 16, caractérisé en ce que ledit site unique est situé dans le gène xylE ou bien en amont de celui-ci et en aval du promoteur.

18) Plasmide vecteur selon l'une des revendications 16 et 17, caractérisé en ce que le site unique est un site BamHI.

19) Souche bactérienne gram + transformée par un plasmide selon l'une des revendications 1 à 18.

20) Catéchol 2,3-oxygénase obtenue par fermentation d'une souche selon la revendication 19.

21) Application d'un plasmide selon l'une des revendications 1 à 18 au clonage d'un gène dans une bactérie gram +.

22) Phage M13 caractérisé en ce que son ADN comporte au moins le gène xylE codant pour la synthèse de la catéchol 2,3-oxygénase.

23) Phage selon la revendication 22, caractérisé en ce qu'il comporte au moins un site unique de restriction situé en un endroit tel que l'insertion d'une séquence d'ADN dans ce site conduise à l'inactivation du gène xylE.

24) Phage selon la revendication 22, caractérisé en ce qu'il comporte un site de clonage multiple placé de façon que l'insertion d'une séquence d'ADN dans un point de ce site inactive l'expression du gène xylE.

25) Phage selon l'une des revendications 22 à 24, caractérisé en ce qu'il s'agit d'un dérivé du phage M13.

26) Application du phage selon l'une des revendications 22 à 25 au clonage et au séquençage d'ADN.

27) Composition pour application topique externe, caractérisée en ce qu'elle comporte de la catéchol 2,3-oxygénase.

28) Composition selon la revendication 27, caractérisée en ce qu'elle comporte, en outre, une base cosmétique.

29) Composition selon la revendication 27, caractérisée en ce qu'elle est destinée au traitement et à la prévention des allergies.

30) Application de la catéchol 2,3-oxygénase à titre d'additif alimentaire.

31) Application de la catéchol 2,3-oxygénase dans le domaine pharmaceutique pour modifier la structure des catécholamines.

32) Composition de décontamination de surface, caractérisée en ce qu'elle comporte une base détergente et une enzyme, la catéchol 2,3-oxygénase.

33) Composition selon la revendication 32, caractérisée en ce que le pH est compris entre 6 et 10.

34) Composition selon la revendication 32, caractérisée en ce qu'elle comprend, en outre, une protéase.

35) Composition selon la revendication 32, caractérisée en ce que la catéchol 2,3-oxygénase est préparée à partir de Bacillus subtilis.

36) Composition selon la revendication 35, caractérisée en ce que la catéchol 2,3-oxygénase est préparée à partir d'un B. subtilis producteur de protéase.

37) Composition selon la revendication 32, caractérisée en ce qu'elle contient un agent de stabilisation.

38) Composition selon la revendication 37, caractérisée en ce que l'agent de stabilisation est l'acétone.

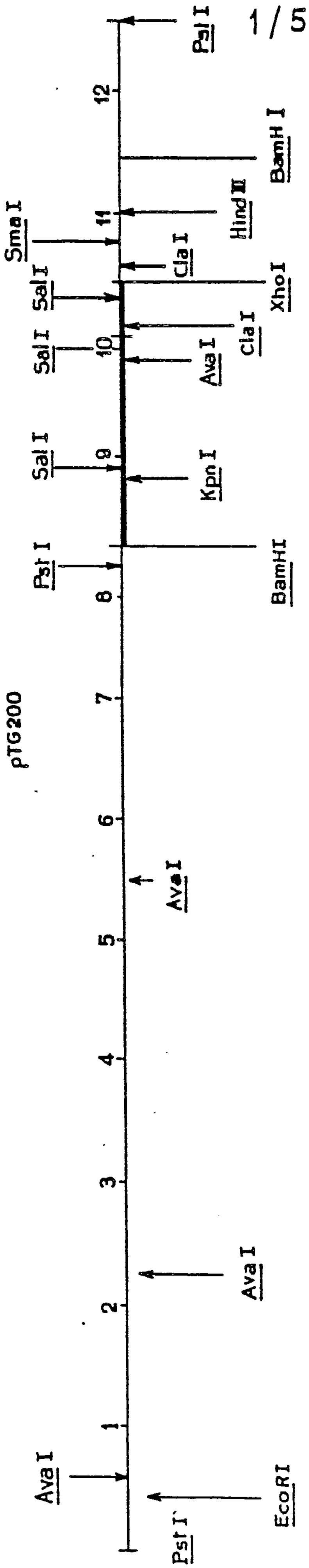
Pst I
12
BamH I
Hind III
Sma I
11
Cla I
Sal I
Xho I
Cla I
Sal I
10
Ava I
Sal I
9
Kpn I
Pst I
BamHI
8
7
pTG200
6
Ava I
5
4
3
Ava I
2
1
Ava I
EcoRI
Pst I

FIG_1

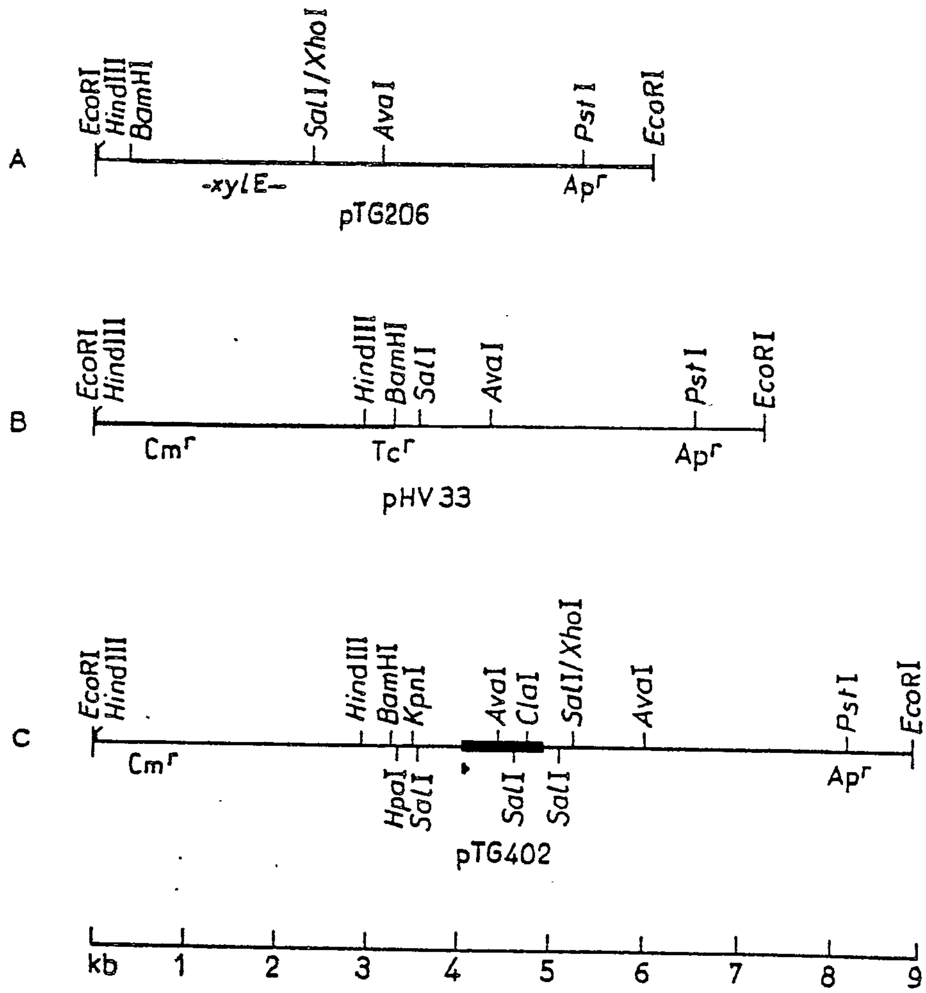
A
EcoRI
HindIII
BamHI
SalI/XhoI
AvaI
PstI
EcoRI
-xylE-
Apr
pTG206
B
EcoRI
HindIII
HindIII
BamHI
SalI
AvaI
PstI
EcoRI
Cmr
Tcr
Apr
pHV33
C
EcoRI
HindIII
HindIII
BamHI
KpnI
AvaI
ClaI
SalI/XhoI
AvaI
PstI
EcoRI
Cmr
HpaI
SalI
SalI
SalI
Apr
pTG402
kb
1
2
3
4
5
6
7
8
9

FIG_2

1 ... 50
GACAACATGAACTATGAAGAGGTGACGTC ATG AAC AAA GGT GTA ATG CGA CCG GGC CAT GTG CAG CTG CGT GTA CTG GAC ATG
Met Asn Lys Gly Val Met Arg Pro Gly His Val Gln Leu Arg Val Leu Asp Met

100
AGC AAG GCC CTG GAA CAC TAC GTC GAG TTG CTG GGC CTG ATC GAG ATG GAC CGT GAC GAC CAG GGC CGT GTC TAT CTG
Ser Lys Ala Leu Glu His Tyr Val Glu Leu Leu Gly Leu Ile Glu Met Asp Arg Asp Asp Gln Gly Arg Val Tyr Leu

150 ... 200
AAG GCT TGG ACC GAA GTG GAT AAG TTT TCC CTG GTG CTA CGC GAG GCT GAC GAG CCG GGC ATG GAT TTT ATG GGT TTC
Lys Ala Trp Thr Glu Val Asp Lys Phe Ser Leu Val Leu Arg Glu Ala Asp Glu Pro Gly Met Asp Phe Met Gly Phe

250
AAG GTT GTG GAT GAG GAT GCT CTC CGG CAA CTG GAG CGG GAT CTG ATG GCA TAT GGC TGT GCC GTT GAG CAG CTA CCC
Lys Val Val Asp Glu Asp Ala Leu Arg Gln Leu Glu Arg Asp Leu Met Ala Tyr Gly Cys Ala Val Glu Gln Leu Pro

300 ... 350
GCA GGT GAA CTG AAC AGT TGT GGC CGG CGC GTG CGT TCC AGG CCC TCC GGG CAT CAC TTC GAG TTG TAT GCA GAC AAG
Ala Gly Glu Leu Asn Ser Cys Gly Arg Arg Val Arg Ser Arg Pro Ser Gly His His Phe Glu Leu Tyr Ala Asp Lys

400
GAA TAT ACT GGA AAG TGG GGT TTG AAT GAC GTC AAT CCC GAG GCA TGG CCG CGC GAT CTG AAA GGT ATG GCG GCT GTG
Glu Tyr Thr Gly Lys Trp Gly Leu Asn Asp Val Asn Pro Glu Ala Trp Pro Arg Asp Leu Lys Gly Met Ala Ala Val

450 ... 500
CGT TTC GAC CAC GCC CTC ATG TAT GGC GAC GAA TTG CCG GCG ACC TAT GAC CTG TTC ACC AAG GTG CTC GGT TTC TAT
Arg Phe Asp His Ala Leu Met Tyr Gly Asp Glu Leu Pro Ala Thr Tyr Asp Leu Phe Thr Lys Val Leu Gly Phe Tyr

550
CTG GCC GAA CAG GTG CTG GAC GAA AAT GGC ACG CGC GTC GCC CAG TTT CTC AGT CTG TCG ACC AAG GCC CAC GAC GTG
Leu Ala Glu Gln Val Leu Asp Glu Asn Gly Thr Arg Val Ala Gln Phe Leu Ser Leu Ser Thr Lys Ala His Asp Val

650
GCC TTC ATT CAC CAT CCG GAA AAA GGC CGC CTC CAT CAT GTG TCC TTC CAC CTC GAA ACC TGG GAA GAC TTG CTT CGC
Ala Phe Ile His His Pro Glu Lys Gly Arg Leu His His Val Ser Phe His Leu Glu Thr Trp Glu Asp Leu Leu Arg

700 ... 750
GCC GCC GAC CTG ATC TCC ATG ACC GAC ACA TCT ATC GAT ATC GGC CCA ACC CGC CAC GGC CTC ACT CAC GGC AAG ACC
Ala Ala Asp Leu Ile Ser Met Thr Asp Thr Ser Ile Asp Ile Gly Pro Thr Arg His Gly Leu Thr His Gly Lys Thr

800
ATC TAC TTC TTC GAC CCG TCC GGT AAC CGC AAC GAA GTG TTC TGC GGG GGA GAT TAC AAC TAC CCG GAC CAC AAA CCG
Ile Tyr Phe Phe Asp Pro Ser Gly Asn Arg Asn Glu Val Phe Cys Gly Gly Asp Tyr Asn Tyr Pro Asp His Lys Pro

850 ... 900
GTG ACC TGG ACC ACC GAC CAG CTG GGC AAA GCC TTC TTT TAC CAC GAC CGC ATT CTC AAC GAA CGA TTC ATG ACC GTG
Val Thr Trp Thr Thr Asp Gln Leu Gly Lys Ala Phe Phe Tyr His Asp Arg Ile Leu Asn Glu Arg Phe Met Thr Val

CTG ACC TGA TGGTCCGG
Leu Thr ***

FIG_3

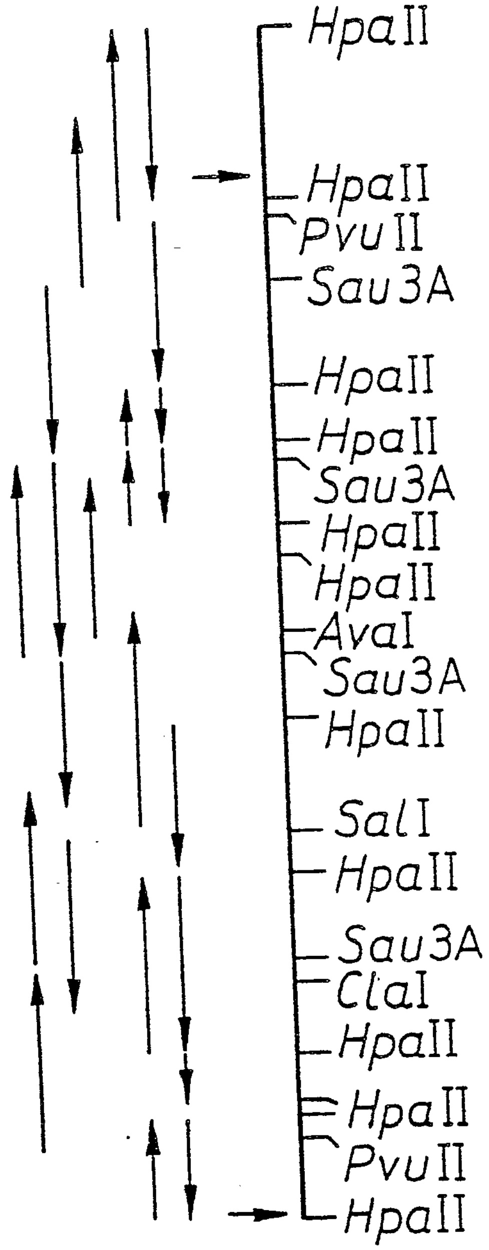
HpaII
HpaII
PvuII
Sau3A
HpaII
HpaII
Sau3A
HpaII
HpaII
AvaI
Sau3A
HpaII
SalI
HpaII
Sau3A
ClaI
HpaII
HpaII
PvuII
HpaII

FIG_4

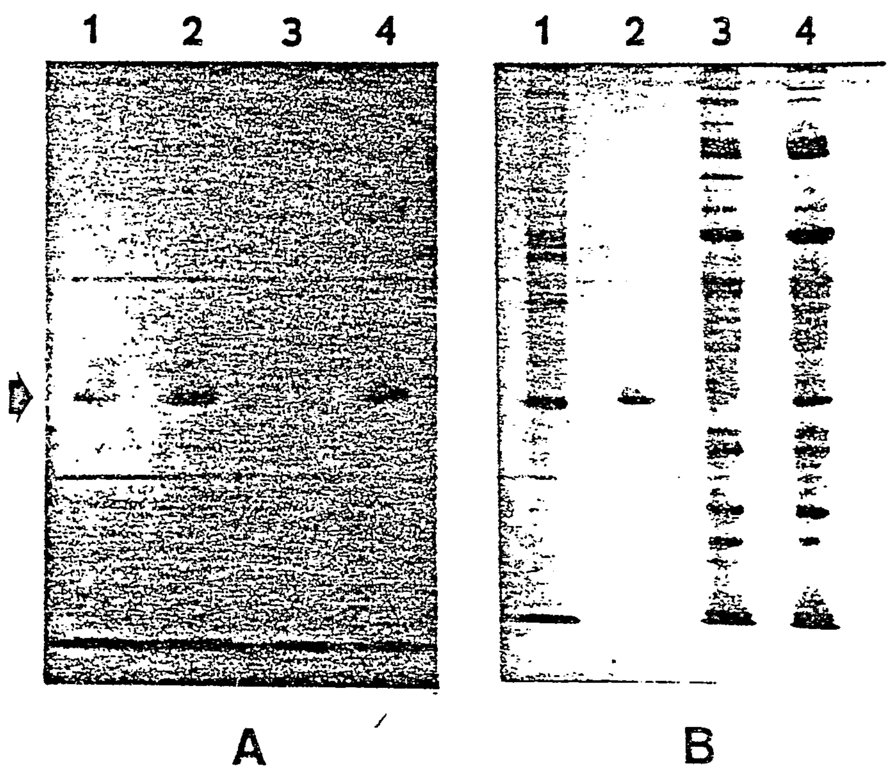


FIG_5